# EUROPEAN PATENT APPLICATION

(11) **EP 2 368 988 A1**
(43) Date of publication of application: **28.09.2011**
(21) Application number: 11152132.4
(22) Date of filing: 19.09.2006
(51) Int. Cl.: C12N 15/11, A61K 31/7088, A61P 3/00, C07H 21/00, A61K 38/00

(54) **Modulation of Glucocorticoid Receptor Expression**

(30) Priority: 19.09.2005 US 718685 P
(62) Divisional of application: 06814959.0
(71) Applicant: Isis Pharmaceuticals, Inc., Carlsbad, CA 92010 (US)
(72) Inventor: Monia, Brett P., Encinitas, CA 92024 (US); McKay, Robert, Poway, CA 92064 (US); Freier, Susan M., San Diego, CA 92122 (US); Bhanot, Sanjay, Carlsbad, CA 92009 (US); Watts, Lynnetta, Carlsbad, CA 92009 (US)
(74) Representative: Hallybone, Huw George

(57) **Abstract**

Compounds, compositions and methods are provided for modulating the expression of glucocorticoid receptor. The compositions comprise antisense compounds, particularly antisense oligonucleotides which have particular in vivo properties, targeted to nucleic acids encoding glucocorticoid receptor. Methods of using these compounds for modulation of glucocorticoid receptor expression and for treatment of diseases are provided.

## Description

### SEQUENCE LISTING

A computer-readable form of the sequence listing, on diskette, containing the file named BIOLOO65WOSEQ.txt, which is 37,122 bytes (measured in MS-DOS) and was created on September 19, 2006, is herein incorporated by reference.

### FIELD OF THE INVENTION

Disclosed herein are compounds, compositions and methods for modulating the expression of glucocorticoid receptor in a cell, tissue or animal.

### BACKGROUND OF THE INVENTION

As increased gluconeogenesis is considered to be the major source of increased glucose production in diabetes, a number of therapeutic targets for the inhibition of hepatic glucose production have been investigated. Due to the ability of antagonists of the glucocorticoid receptor (also known as nuclear receptor subfamily 3, group C, member1; NR3C1; GCCR; GCR; GRL; Glucocorticoid receptor, lymphocyte) to ameliorate diabetes in animal models, such compounds are among the potential therapies being explored. However, there are detrimental systemic effects of glucocorticoid receptor antagonists;-- - - -- - including activation of the HPA axis (Link, Curr Opin Investig Drugs, 2003, 4, 421-429). Increased SPA axis activity is associated with suppression of immune-related inflammatory action, which can increase susceptibility to infectious agents and neoplasms. Conditions associated with suppression of immune-mediated inflammation through defects in the HPA axis, or its target tissues, include Cushing's syndrome, chronic stress, chronic alcoholism and melancholic depression (Chrousos, N Engl J Med, 1995, 332, 1351-1362). Thus, it is of great value to develop liver and fat-specific glucocorticoid receptor antagonists.

### SUMMARY OF THE INVENTION

The present invention is directed to oligomeric compounds targeted to and hybridizable with a nucleic acid molecule encoding GCCR which modulate the expression of GCCR. Provided herein are chimeric oligonucleotides referred to as "gapmers", comprising a deoxynucleotide region or "gap" flanked on each of the 5' and 3' ends with "wings" comprised of one to four 2'-O-methoxyethyl nucleotides. The deoxynucleotide regions of the oligonucleotides of the invention are comprised of greater than ten deoxynucleotides, thus the gapmers of the present invention are "gap-widened" as compared to chimeric compounds comprising a ten deoxynucleotide gap region, such as are exemplified in US Publication US2005-0164271, which is herein incorporated by reference in its entirety. In some embodiments, as compared to oligonucleotides having the same sequence but comprising a ten deoxynucleotide region flanked on both the 5' and 3' ends with five 2'-O-(2-methoxyethyl) nucleotides, gap-widened oligonucleotides have comparable or improved potency without enhanced accumulation of oligonucleotide in the liver. Thus, embodiments of th- present invention include gap-widened oligonucleotides targeting GCCR wherein potency is comparable to or better than that of an oligonucleotide having the same sequence but comprising a ten deoxynucleotide region flanked on both the 5' and 3' ends with five 2'-O-(2-methoxyethyl) nucleotides without enhanced accumulation of oligonucleotide in target tissues.

Another embodiment of the present invention includes gap-widened oligonucleotides targeting GCCR wherein kidney concentrations of said oligonucleotide are comparable to or decreased with respect to that of an oligonucleotide having the same sequence but comprising a ten deoxynucleotide region flanked on both the 5' and 3' ends with five 2'-O-(2-methoxyethyl) nucleotides while maintaining or improving potency in target tissues such as liver.

Further provided are methods of modulating the expression of GCCR in cells, tissues or, animals comprising contacting said cells, tissues or animals with one or more of the compounds or compositions of the present invention. For example, in one embodiment, the compounds or compositions of the present invention can be used to reduce the expression of GCCR in cells, tissues or animals.

In one embodiment, the present invention provides a method of treating a disease or condition mediated by glucocorticoid expression in an animal comprising contacting the animal with an effective amount of a compound of the invention. The diseases or conditions include diabetes, Type 2 diabetes, obesity, metabolic syndrome X, hyperglycemia, hyperlipidemia, or liver steatosis. In some embodiments, the hyperlipidemia is associated with elevated lipids such as blood cholesterol or elevated blood triglycerides. Blood lipids include plasma lipids and serum lipids. Further provided are methods of decreasing blood lipid levels, methods of reducing body fat mass, methods of decreasing liver triglyceride levels, and methods of improving insulin sensitivity in an animal by administering a compound of the invention.

Also provided is a method of decreasing blood glucose levels in an animal comprising administering a compound of the invention. The blood glucose levels may be fasting or fed glucose levels, and blood glucose levels include serum or plasma glucose levels. Further provided are methods of increasing insulin sensitivity and inhibiting hepatic glucose output.

Another aspect of the present invention is a method of delaying or preventing the onset of an increase in blood lipid or blood glucose levels in an animal by administering a compound of the invention.

The instant application is also related to U.S. Application No. 60/718,684, which is incorporated by reference in its entirety. The instant application is also related to tT.S. Application No. 11/231,243 and PCT Application No. PCTIUS2005/033837, each of which is herein incorporated by reference in its entirety.

### DETAILED DESCRIPTION:

### Overview

Disclosed herein are oligomeric compounds, including antisense oligonucleotides and other antisense compounds for use in modulating the expression of nucleic acid molecules encoding GCCR. This is accomplished by providing oligomeric compounds which hybridize with one or more target nucleic acid molecules encoding GCCR.

In accordance with the present invention are compositions and methods for modulating the expression of GCCR (also known as glucocorticoid receptor; nuclear receptor subfamily 3, group C, member I; GR; GRL; and NR3C1). Listed in Table 1 are GENBANK® accession numbers of sequences which may be used to design oligomeric compounds targeted to GCCR. Oligomeric compounds of the invention include oligomeric compounds which hybridize with one or more target nucleic acid molecules shown in Table 1, as well as oligomeric compounds which hybridize to other nucleic acid molecules encoding GCCR.

The oligomeric compounds may target any region, segment, or site of nucleic acid molecules which encode GCCR. Suitable target regions, segments, and sites include, but are not limited to, the 5'UTR, the start codon, the stop codon, the coding region, the 3'UTR, the 5'cap region, introns, exons, intron-exon junctions, exon-intron junctions, and exon-exon junctions.

**Table 1**

| **Gene Targets** | | |
|---|---|---|
| **Species** | **GENBANK® Accession Number or Description** | **SEQ ID NO** |
| Human | NM 000176.1 | 1 |
| Mouse | NM _012576.1 | 2 |
| Rat | NM_008173.1 | 3 |

The locations on the target nucleic acid to which active oligomeric compounds hybridize are herein below referred to as "validated target segments." As used herein the term "validated target segment" is defmed as at least an 8-nucleobase portion of a target region to which an active oligomeric compound is targeted. While not wishing to be bound by theory, it is presently believed that these target segments represent portions of the target nucleic acid which are accessible for hybridization.

The present invention includes oligomeric compounds which are chimeric compounds. An example of a chimeric compound is a gapmer having a 2'-deoxynucleotide region or "gap" flanked by non-deoxynucleotide regions or "wings". While not wishing to be bound by theory, the gap of the gapmer presents a substrate recognizable by RNase H when bound to the RNA target whereas the wings are not an optimal substrate but can confer other properties such as contributing to duplex stability or advantageous pharmacokinetic effects. Each wing can be one or more non-deoxy oligonucleotide monomers. In one embodiment, the gapmer is comprised of a sixteen 2'-deoxynucleotide region flanked on each of the 5' and 3' ends by wings of two 2'-O-(2-methoxyethyl) nucleotides. This is referred to as a 2-16-2 gapmer. Thus, the "motif of this chimeric oligomeric compound or gapmer is 2-16-2. ill another embodiment, all of the internucleoside linkages are phosphorothioate linkages. In another embodiment the cytosines of the gapmer are 5-methylcytosines.

Embodiments of the present invention include oligomeric compounds comprising sequences of 13 to 26 nucleotides in length comprising a deoxy nucleotide region greater than 10 nucleobases in length flanked on each of its 5' and 3' ends with at least one 2'-O-(2-methoxyethyl) nucleotide. The preferred "gap-widened" oligonucleotides comprise 11, 12, 13, 14, 15, 16, 17, or 18 deoxynucleotides in the gap portion of the oligonucleotide. Preferred 5' and 3' flanking regions comprise 1, 2, 3, or 4 2'-O-(2-methoxyethyl) nucleotides. Preferred gap-widened gapmers have motifs including 1-18-1, 1-17-2, 2-17-1, 2-16-2, 3-14-3, and 4-12-4.

In preferred embodiments the oligomeric compounds target or hybridize with GCCR RNA. In another embodiment, the oligomeric compounds reduce the expression of GCCR RNA. In other embodiments, the oligomeric compounds reduce the expression of GCCR wherein the expression of GCCR is reduced by at least 10%, by at least 20%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, or by 100%.

Oligonucleotides of the present invention include those wherein kidney concentrations of said oligonucleotide are decreased with respect to an oligonucleotide having the same sequence but comprising a ten deoxynucleotide region flanked on both the 5' and 3' ends with five 2'-O-(2-methoxyethyl) nucleotides. Oligonucleotides of the present invention include those wherein kidney concentrations of said oligonucleotide are comparable to or decreased with respect to those of an oligonucleotide having the same sequence but comprising a ten deoxynucleotide region flanked on both the 5'- and 3' ends with five 2'-O-(2-methoxyethyl) nucleotides. Oligonucleotides of the present invention include those wherein potency with regard to target reduction or a therapeutic effect is comparable to or better than that of an oligonucleotide having the same sequence but comprising a ten deoxynucleotide region flanked on both the 5' and 3' ends with five 2'-O-(2-methoxyethyl) nucleotides without enhanced accumulation of oligonucleotide in target tissues. Preferred target tissues include liver, and adipose tissue.

The present invention provides antisense oligonucleotides 13 to 26 nucleobases in length targeted to a nucleic acid molecule encoding GCCR wherein the oligonucleotide comprises a first region, a second region, and a third region, wherein said first region comprises at least 11 deoxynucleotides and wherein said second and third regions comprise 1 to 4 2'-O-(2-methoxyethyl) nucleotides, said second and third regions flanking the first region on the 5' and 3' ends of said first region.

In some embodiments, oligonucleotides of the invention specifically hybridize to GCCR and reduce expression of GCCR. In some embodiments, the "gap" region comprises 11,12,13,14,15,16, 17, or 18 nucleobases. In some embodiments, the antisense oligonucleotides are 20 nucleobases in length.

The oligomeric compounds can comprise about 8 to about 80 nucleobases (i.e. from about 8 to about 80 linked nucleosides), preferably between about 13 to about 26 nucleobases. One of ordinary skill in the art will appreciate that the preferred oligomeric compounds contemplated include compounds that are 13, 14, 15, 16, 17, 18, 19, 20, 21, 22, 23, 24, 25, or 26 nucleobases in length.

Compounds of the invention include oligonucleotide sequences that comprise at least the 8 consecutive nucleobases from the 5'-terminus of one of the illustrative antisense compounds (the remaining nucleobases being a consecutive stretch of the same oligonucleotide beginning immediately upstream of the 5'-tetxninus of the antisense compound which is specifically hybridizable to the target nucleic acid and continuing until the oligonucleotide comprises about 13 to about 26 nucleobases). Other compounds are represented by oligonucleotide sequences that comprise at least the 8 consecutive nucleobases from the 3''-terminus of one of the illustrative antisense compounds (the remaining nucleobases being a consecutive stretch of the same oligonucleotide beginning immediately downstream of the 3'-tertninus of the antisense compound which is specifically hybridizable to the target nucleic acid and continuing until the oligonucleotide comprises about 13 to about 26 nucleobases). It is also understood that compounds may be represented by oligonucleotide sequences that comprise at least 8 consecutive nuc1eobases from an internal portion of the sequence of an illustrative compound, and may extend in either or both directions until the oligonucleotide contains about 13 to about 26 nucleobases.

Oligonucleotides of the invention include antisense oligonucleotides 20 nucleobases in length targeted to a nucleic acid molecule encoding GCCR and comprising at least an 8-nucleobase portion of SEQ ID NO: 34, 33, 35; 36, 37, 42, 45, 56, 61, 63, or 96. In one embodiment, oligonucleotides of the invention are antisense oligonucleotides 20 nucleobases in length targeted to a nucleic acid molecule encoding GCCR and having the sequence of SEQ II7 NO: 34, 33,35,36,37,42,45,56, 61, 63, or 96. In one embodiment, oligonucleotides of the invention have the nucleobase sequence of SEQ m N9 : 37..

The present invention provides antisense oligonucleotides comprising the nucleobase sequence of SEQ ill NO: 37. In one embodiment, the oligonucleotides of the invention comprise at least an 8-nucleobase portion of the nucleobase sequence of SEQ ID NO: 37.

In one embodiment, the present invention provides antisense oligonucleotides 20 nucleobases in length targeted to a nucleic acid molecule encoding GCCR and comprising at least an 8 nucleobase portion of SEQ ID NO: 34, 33, 35, 36, 37, 42, 45, 56, 61, 63, or 96 wherein the oligonucleotide comprises a deoxynucleotide region 12, 13, 14, 15, 16, 17, or 18 nucleobases in length which is flanked on its 5' and 3' ends with 1 to 4 2'-O-(2-methoxyethyl) nucleotides and wherein the oligonucleotide specifically hybridizes to and reduces expression of GCCR RNA.

In one embodiment, the flanking regions are symmetrical (having the same number of nucleotides in the 5' flanking region as in the 3' flanking region). In another embodiment, the flanking regions are non-symmetrical (having a different number of nucleotides in the S' flanking region compared to the 3' flanking region).

Antisense oligonucleotides of the invention may contain at least one modified internucleoside linkage. Modified internucleoside linkages include phosphorothioate linkages. The antisense oligonucleotides of the invention may also contain at least one modified nucleobase. In preferred embodiments, at least one cytosine is a 5-methylcytosine.

In other embodiments, the present invention includes antisense oligonucleotides having the nucleobase sequence of SEQ ID NO: 37, wherein the antisense oligonucleotide is characterized by a 12-deoxynucleotide region flanked on its 5' and 3' ends with four 2'-O-(2-methoxyethyl) nucleotides, a 14-deoxynucleotide region flanked on its 5' and 3' ends with three 2'-O-(2-methoxyethyl) nucleotides, a 16-deoxynucleotide region flanked on its 5' and 3' ends with two 2'-O-(2-ixzethoxyethyl) nucleotides, a 17-deoxynucleotide region flanked on its 5' and 3' ends with one or two 2'-O-(2-methoxyethyl) nucleotides, or an 18-deoxynucleotide region flanked on its 5' and 3' ends with one 2'-O-(2-methoxyethyl) nucleotides.

In a particular embodiment, the antisense oligonucleotides have the nucleobase sequence of SEQ ID: 37, wherein the antisense oligonucleotide has a 12-deoxynucleotide region flanked on its 5' and 3' ends with four 2'-O(2-methoxyethyl) nucleotides. In a further embodiment, the antisense oligonucleotide specifically hybridizes to and reduces expression of GCCR. In a further embodiment, at least one internucleoside linkage is a phosphorothioate linkage. In a further embodiment, at least one cytosine is a 5-methylcytosine.

In a particular embodiment, the antisense oligonucleotide has the nucleobase sequence of SEQ ID: 37, wherein the antisense oligonucleotide has a 14-deoxynucleotide region flanked on its 5' and 3' ends with three 2'-O(2-methoxyethyl) nucleotides. In a further embodiment, the antisense oligonucleotide specifically hybridizes to and reduces expression of GCCR. In a further embodiment, at least one internucleoside linkage is a phosphorothioate linkage. In a further embodiment, at least one cytosine is a 5-methylcytosine.

In a particular embodiment, the antisense oligonucleotide has the nucleobase sequence of SEQ ID: 37, wherein the antisense oligonucleotide has a 16-deoxynucleotide region flanked on its 5' and 3' ends with two 2'-O(2-methoxyethyl) nucleotides. In a further embodiment, the antisense oligonucleotide specifically hybridizes to and reduces expression of GCCR. In a further embodiment, at least one internucleoside linkage is a phosphorothioate linkage. In a further embodiment, at least one cytosine is a 5-methylcytosine.

In a particular embodiment, the antisense oligonucleotide has the nucleobase sequence of SEQ 3i7i: 37, wherein the antisense oligonucleotide has a 17-deoxynucleotide region flanked on its 5' and 3' ends with one or two 2'-O(2-methoxyethyl) nucleotides. In a further embodiment, the antisense oligonucleotide specifically hybridizes to and reduces expression of GCCR In a further embodiment, at least one internucleoside linkage is a phosphorothioate linkage. In a further embodiment, at least one cytosine is a 5-methylcytosine.

In a particular embodiment, the antisense oligonucleotide has the nucleobase sequence of SEQ ID: 37, wherein the antisense oligonucleotide has a 18-deoxynucleotide region flanked on its 5' and 3' ends with one 2'-O(2-methoxyethyl) nucleotides. In a further embodiment, the antisense oligonucleotide specifically hybridizes to and reduces expression of GCCR. In a further embodiment, at least one internucleoside linkage is a phosphorothioate linkage. In a further embodiment, at least one cytosine is a 5-methylcytosine.

In another embodiment the antisense oligonucleotides comprise the nucleobase sequence of SEQ ID NO: 33. In one embodiment, the oligonucleotides of the invention comprise at least an 8-nucleobase portion of the nucleobase sequence of SEQ ID NO: 33.

In other embodiments, the present invention includes antisense oligonucleotides having the nucleobase sequence of SEQ ID NO: 33, wherein the antisense oligonucleotide is characterized by a 12-deoxynucleotide region flanked on its 5' and 3' ends with four 2'-O-(2-methoxyethyl) nucleotides, a 14-deoxynucleotide region flanked on its 5' and 3' ends with three 2'-O-(2-methoxyethyl) nucleotides, a 16-deoxynucleotide region flanked on its 5' and 3' ends with two 2'-O-(2-methoxyethyl) nucleotides, a 17-deoxynucleotide region flanked on its 5' and 3' ends with one or two 2'-O-(2-methoxyethyl) nucleotides, or an 18-deoxynucleotide region flanked on its 5' and 3' ends with one 2'-O-(2-methoxyethyl) nucleotides.

In a particular embodiment, the antisense oligonucleotides have the nucleobase sequence of SEQ ID: 33, wherein the antisense oligonucleotides have a 12-deoxynucleotide region flanked on its 5' and 3' ends with four 2'-O(2-methoxyethyl) nucleotides. In a further embodiment, the antisense oligonucleotide specifically hybridizes to and reduces expression of GCCR. In a further embodiment, at least one internucleoside linkage is a phosphorothioate linkage. In a further embodiment, at least one cytosine is a 5-methylcytosine.

In a particular embodiment, the antisense oligonucleotide has the nucleobase sequence of SEQ ID: 33, wherein the antisense oligonucleotide has a 14-deoxynucleotide region flanked on its 5' and 3' ends with three 2'-O(2-methoxyethyl) nucleotides. In a further embodiment, the antisense oligonucleotide specifically hybridizes to and reduces expression of GCCR. In a further embodiment, at least one internucleoside linkage is a phosphorothioate linkage. In a further embodiment, least one cytosine is a 5-methylcytosine.

In a particular embodiment, the antisense oligonucleotide has the nucleobase sequence of SEQ 1D: 33, wherein the antisense oligonucleotide has a 16-deoxynucleotide region flanked on its 5' and 3' ends with two 2'-O(2-methoxyethyl) nucleotides. In a further embodiment, the antisense oligonucleotide specifically hybridizes to and reduces expression of GCCR. In a further embodiment, at least one internucleoside linkage is a phosphorothioate linkage. In a further embodiment, at least one cytosine is a 5-methylcytosine.

In a particular embodiment, the antisense oligonucleotide has the nucleobase sequence of SEQ ID: 33, wherein the antisense oligonucleotide has a 17-deoxynucleotide region flanked on its 5' and 3' ends with one or two 2'-O(2-methoxyethyl) nucleotides. In a further embodiment, the antisense oligonucleotide specifically hybridizes to and reduces expression of GCCR. In a further embodiment, at least one internucleoside linkage is a phosphorothioate linkage. In a further embodiment, at least one cytosine is a 5-methylcytosine.

In a particular embodiment, the antisense oligonucleotide has the nucleobase sequence of SEQ ID: 33, wherein the antisense oligonucleotide has a 18-deoxynucleotide region flanked on its 5' and 3' ends with one 2'-O(2-methoxyethyl) nucleotides. In a further embodiment, the antisense oligonucleotide specifically hybridizes to and reduces expression of GCCR. In a further embodiment, at least one internucleoside linkage is a phosphorothioate linkage. In a further embodiment, at least one cytosine is a 5-methylcytosine.

The present invention provides antisense oligonucleotides comprising the nucleobase sequence of SEQ 1D NO: 45. In one embodiment, the oligonucleotides of the invention comprise at least an 8-nucleobase portion of the nucleobase sequence of SEQ ID NO: 45.

In other embodiments, the present invention includes antisense oligonucleotides having the nucleobase sequence of SEQ ID NO: 45, wherein the antisense oligonucleotide is characterized by a 12-deoxynucleotide region flanked on its 5' and 3' ends with four 2'-O-(2-methoxyethyl) nucleotides, a 14-deoxynucleotide region flanked on its 5' and 3' ends with three 2'-O-(2-methoxyethyl) nucleotides, a 16-deoxynucleotide region flanked on its 5' and 3' ends with two 2'-O-(2-methoxyethyl) nucleotides, a 17-deoxynucleotide region flanked on its 5' and 3' ends with one or two 2'-O-(2-methoxyethyl) nucleotides, or an 18-deoxynucleotide region flanked on its 5' and 3' ends with one 2'-O-(2-methoxyethyl) nucleotides.

In a particular embodiment, the antisense oligonucleotides have the nucleobase sequence of SEQ ID: 45, wherein the antisense oligonucleotides have a 12-deoxynucleotide region flanked on its 5' and 3' ends with four 2'-O(2-methoxyethyl) nucleotides. In a further embodiment, the antisense oligonucleotide specifically hybridizes to and reduces expression of GCCR. In a further embodiment, at least one internucleoside linkage is a phosphorothioate linkage. In a further embodiment, at least one cytosine is a 5-methylcytosine.

In a particular embodiment, the antisense oligonucleotide has the nucleobases sequence of SEQ ID: 45, wherein the antisense oligonucleotide has a 14-deoxynucleotide region flanked on its 5' and 3' ends with three 2'-O(2-methoxyethyl) nucteotides. In a further embodiment, the antisense oligonucleotide specifically hybridizes to and reduces expression of GCCR. In a further embodiment, at least one internucleoside linkage is a phosphorothioate linkage, In a further embodiment, least one cytosine is a 5-methylcytosine.

In a particular embodiment, the antisense oligonucleotide has the nucleobase sequence of SEQ ID: 45, wherein the antisense oligonucleotide has a 16-deoxynucleotide region flanked on its 5' and 3' ends with two 2'-O(2-methoxyethyl) nucleotides. In a further embodiment, the antisense oligonucleotide specifically hybridizes to and reduces expression of GCCR. In a further embodiment, at least one internucleoside linkage is a phosphorothioate linkage. In a further embodiment, at least one cytosine is a 5-methylcytosine.

In a particular embodiment, the antisense oligonucleotide has the nucleobase sequence of SEQ ID: 45, wherein the antisense oligonucleotide has a 17-deoxynucleotide region flanked on its 5' and 3' ends with one or two 2'-O(2-methoxyethyl) nucleotides. In a further embodiment, the antisense oligonucleotide specifically hybridizes to and reduces expression of GCCR. In a further embodiment, at least one intemacleoside linkage is a phosphorothioate linkage. In a further embodiment, at least one cytosine is a 5-methylcytosine.

In a particular embodiment, the antisense oligonucleotide has the nucleobase sequence of SEQ 1D: 45, wherein the antisense oligonucleotide has a 18-deoxynucleotide region flanked on its 5' and 3' ends with one 2'-O(2-methoxyethyl) nucleotides. In a further embodiment, the antisense oligonucleotide specifically hybridizes to and reduces expression of GCCR. In a further embodiment, at least one internucleoside linkage is a phosphorothioate linkage. In a further embodiment, at least one cytosine is a 5-methylcytosine.

Also contemplated herein is a pharmaceutical composition comprising an antisense oligonucleotide of the invention and optionally a pharmaceutically acceptable carrier, diluent, enhancer or excipient. The compounds of the invention can also be used in the manufacture of a medicament for the treatment of diseases and disorders related to glucocorticoid activity mediated by GCCR.

Embodiments of the present invention include methods of reducing the expression of GCCR in tissues or cells comprising contacting said cells or tissues with a pharmaceutical composition or an antisense oligonucleotide of the invention, methods of decreasing blood glucose levels, blood triglyceride levels, or blood cholesterol levels in an animal comprising administering to said animal a pharmaceutical composition of the invention. Blood levels may be plasma or serum levels. Also contemplated are methods of increasing insulin sensitivity, methods of decreasing liver triglyceride levels, and methods of inhibiting hepatic glucose output in an animal comprising administering to said animal a pharmaceutical composition of the invention. Increased insulin sensitivity may be indicated by a decrease in circulating insulin levels. Another aspect of the present invention is a method of reducing body fat mass in an animal.

Other embodiments of the present invention include methods of treating an animal having a disease or condition associated with glucocorticoid receptor expression comprising administering to said animal a therapeutically or prophylactically effective amount of an antisense oligonucleotide of the invention. The disease or condition may be a metabolic disease or condition. In some embodiments, the metabolic disease or condition is diabetes, obesity, metabolic syndrome X, hyperglycemia, hyperlipidemia, or insulin resistance. In some embodiments, the disease is Type 2 diabetes. In some embodiments, the obesity is diet-induced. In some embodiments the hyperlipidemia is associated with elevated blood lipid levels. Lipids include cholesterol and triglycerides. In one embodiment, the condition is liver steatosis. In some embodiments, the steatosis is steatohepatitis or non-alcoholic steatohepatitis.

Also provided are methods of preventing or delaying the onset of elevated blood glucose or blood lipid levels in an animal.

Compounds of the invention can be used to modulate the expression of GCCR in an animal in need thereof, such as a human. In one non-limiting embodiment, the methods comprise the step of administering to said animal an effective amount of an antisense compound that reduces expression of GCCR. In one embodiment, the antisense compounds of the present invention effectively reduce the levels or function of GCCR RNA. Because reduction in GCCR mRNA levels can lead to alteration in GCCR protein products of expression as well, such resultant alterations can also be measured. Antisense compounds of the present invention that effectively reduce the levels or function of an GCCR RNA or protein products of expression is considered an active antisense compound. In one embodiment, the antisense compounds of the invention reduce the expression of GCCR causing a reduction of RNA by at least 10%, by at least 20%, by at least 25%, by at least 30%, by at least 40%, by at least 50%, by at least 60%, by at least 70%, by at least 75%, by at least 80%, by at least 85%, by at least 90%, by at least 95%, by at least 98%, by at least 99%, or by 100% as measured by an exemplified assay herein.

### Antisense mechanism

"Antisense mechanisms" are all those involving hybridization of a compound with target nucleic acid, wherein the outcome or effect of the hybridization is either target degradation or target occupancy with concomitant stalling of the cellular machinery involving, for example, transcription or splicing.

### Targets

_{I}

As used herein, the terms "target nucleic acid" and "nucleic acid molecule encoding GCCR" have been used for convenience to encompass DNA encoding GCCR, RNA (including pre-mRNA and RNA or portions thereof) transcribed from such DNA, and also cDNA derived from such RNA.

### Regions, Segments, and Sites

The targeting process usually also includes determination of at least one target region, segment, or site within the target nucleic acid for the antisense interaction to occur such that the desired effect, e.g., _ modulation of expression, will result. "Region" is defined as a portion of the target nucleic acid having at least one identifiable *structure,* function, or characteristic. Within regions of target nucleic acids are segments. "Segments" are defined as smaller or sub-portions of regions within a target nucleic acid. "Sites," as used in the present invention, are defined as unique nucleobase positions within a target nucleic acid.

Once one or more target regions, segments or sites have been identified, oligomeric compounds are designed which are sufficiently complementary to the target, i.e., hybridize sufficiently well and with sufficient specificity, to give the desired effect.

### Variants

It is also known in the art that alternative RNA transcripts can be produced from the same genomic region of DNA. These alternative transcripts are generally known as "variants." More specifically, "pre-rnl2NA variants" are transcripts produced from the same genomic DNA that differ from other transcripts produced from the same genomic DNA in either their start or stop position and contain both intronic and exonic sequence.

Upon excision of one or more exon or intron regions, or portions thereof during splicing, pre-mRNA variants produce smaller "mRNA variants." Consequently, mRNA variants are processed pre-mRNA variants and each unique pre-mRNA variant must always produce a unique mRNA variant as a result of splicing. These mRNA variants are also known as "alternative splice variants." If no splicing of the pre-mRNA variant occurs then the pre-mRNA variant is identical to the mRNA variant.

It is also known in the art that variants can be produced through the use of alternative signals to start or stop transcription and that pre-mRNAs and mRNAs can possess more that one start codon or stop codon. Variants that originate from a pre-mRNA or mRNA that use alternative start codons are known as "alternative start variants" of that pre-mRNA or mRNA. Those transcripts that use an alternative stop codon are known as "alternative stop variants" of that pre-mRNA or mRNA. One specific type of alternative stop variant is the "polyA variant" in which the multiple transcripts produced result from the alternative selection of one of the "polyA stop signals" by the transcription machinery, thereby producing transcripts that terminate at unique polyA sites. Consequently, the types of variants described herein are also suitable target nucleic acids.

### Modulation of Target Expression

"Modulation" means a perturbation of function, for example, either an increase (stimulation or induction) or a decrease (inhibition or reduction) in expression. As another example, modulation of expression can include perturbing splice site selection of pre-mRNA processing. "Expression" includes all the functions by which a gene's coded information is converted into structures present and operating in a cell. These structures include the products of transcription and translation. "Modulation of expression" means the perturbation of such functions. "Modulators" are those compounds that modulate the expression of GCCR and which comprise at least an 8-nucleobase portion which is complementary to a validated target segment.

Modulation of expression of a target nucleic acid can be achieved through alteration of any number of nucleic acid (DNA or RNA) functions. The functions of DNA to be modulated can include replication and transcription. Replication and transcription, for example, can be from an endogenous cellular template, a vector, a plasmid construct or otherwise. The functions of RNA to be modulated can include translocation functions, which include, but are not limited to, translocation of the RNA to a site of protein translation, translocation of the RNA to sites within the cell which are distant from the site of RNA synthesis, and translation of protein from the RNA. RNA processing functions that can be modulated include, but are not limited to, splicing of the RNA to yield one or more RNA species, capping of the RNA, 3' maturation of the RNA and catalytic activity or complex formation involving the RNA which may be engaged in or facilitated by the RNA. Modulation of expression can result in the increased level of one or more nucleic acid species or the decreased level of one or more nucleic acid species, either temporally or by net steady state level. One result of such interference with target nucleic acid function is modulation of the expression of GCCR. Thus, in one embodiment modulation of expression can mean increase or decrease in target RNA or protein levels. In another embodiment modulation of expression can mean an increase or decrease of one or more RNA splice products, or a change in the ratio of two or more splice products.

### Hybridization and Complementarity

"Hybridization" means the pairing of complementary strands of oligomeric compounds. While not limited to a particular mechanism, the most common mechanism of pairing involves hydrogen bonding, which may be Watson-Crick, Hoogsteen or reversed Hoogsteen hydrogen bonding, between complementary nucleoside or nucleotide bases (nucleobases) of the strands of oligomeric compounds. For example, adenine and thymine are complementary nucleobases which pair through the formation of hydrogen bonds. Hybridization can occur under varying circumstances. An oligomeric compound is specifically hybridizable when there is a sufficient degree of complementarity to avoid non-specific binding of the oligomeric compound to non-target nucleic acid sequences under conditions in which specific binding is desired, i.e., under physiological conditions in the case of *in vivo* assays or therapeutic treatment, and under conditions in which assays are performed in the case of *in vitro* assays.

"Stringent hybridization conditions" or "stringent conditions" refer to conditions under which an oligomeric compound will hybridize to its target sequence, but to a minimal number of other sequences. Stringent conditions are sequence-dependent and will be different in different circumstances, and "stringent conditions" under which oligomeric compounds hybridize to a target sequence are determined by the nature and composition of the oligomeric compounds and the assays in which they are being investigated.

"Complementarity," as used herein, refers to the capacity for precise pairing between two nucleobases on one or two oligomeric compound strands. For example, if a nucleobase at a certain position of an antisense compound is capable of hydrogen bonding with a nucleobase at a certain positions of a target nucleic acid, then the position of hydrogen bonding between the oligonucleotide and the target nucleic acid is considered to be a complementary position. The oligomeric compound and the further DNA or RNA are complementary to each other when a sufficient number of complementary positions in each molecule are occupied by nucleobases which can hydrogen bond with each other. Thus, "specifically hybridizable" and "complementary" are terms which are used to indicate a sufficient degree of precise pairing or complementarity over a sufficient number of nucleobases such that stable and specific binding occurs between the oligomeric compound and a target nucleic acid.

It is understood in the art that the sequence of an oligomeric compound need not be 100% complementary to that of its target nucleic acid to be specifically hybridizable. Moreover, an oligonucleotide may hybridize over one or more segments such that intervening or adjacent segments are not involved in the hybridization event (e.g., a loop structure, mismatch or hairpin structure). The oligomeric compounds of the present invention comprise at least 70%, or at least 75%, or at least 80%, or at least 85%, or at least 90%, or at least 92%, or at least 95%, or at least 97%, or at least 98%, or at least 99% sequence complementarity to a target region within the target nucleic acid sequence to which they are targeted. For example, an oligomeric compound in which 18 of 20 nucleobases of the antisense compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. As such, an oligomeric compound which is 18 nucleobases in length having 4 (four) noncomplementary nucleobases which are flanked by two regions of complete complementarity with the target nucleic acid would have 77.8% overall complementarity with the target nucleic acid and would thus fall within the scope of the present invention. Percent complementarity of an oligomeric compound with a region of a target nucleic acid can be determined routinely using BLAST programs (basic local alignment search tools) and PowerBLAST programs known in the art (Altschul et al., J. Mol. Biol., 1990, 215, 403-410; Zhang and Madden, Genome Res., 1997, 7, 649-656). Percent homology, sequence identity or complementarity, can be determined by, for example, the Gap program (Wisconsin Sequence Analysis Package, Version 8 for Unix, Genetics Computer Group, University Research Park, Madison WI), using default settings, which uses the algorithm of Smith and Waterman (Adv. Appl. Math., 1981, 2, 482-489).

### Oligomeric corn pounds

The term "oligomeric compound" refers to a polymeric structure capable of hybridizing to a region of a nucleic acid molecule. This term includes oligonucleotides, oligonucleosides, oligonucleotide analogs, oligonucleotide mimetics and chimeric combinations of these. Oligomeric compounds are routinely prepared linearly but can be joined or otherwise prepared to be circular. Moreover, branched structures are known in the art. An "antisense compound" or "antisense oligomeric compound" refers to an oligomeric compound that is at least partially complementary to the region of a nucleic acid molecule to which it hybridizes and which modulates (increases or decreases) its expression. Consequently, while all antisense compounds can be said to be oligomeric compounds, not all oligomeric compounds are antisense compounds. An "antisense oligonucleotide" is an antisense compound that is a nucleic acid-based oligomer. An antisense oligonucleotide can be chemically modified. Nonlimiting examples of oligomeric compounds include primers, probes, antisense compounds, antisense oligonucleotides, external guide sequence (EGS) oligonucleotides, alternate splicers, and siRNAs. As such, these compounds can be introduced in the form of single-stranded, double-stranded, circular, branched or hairpins and can contain structural elements such as internal or terminal bulges or loops. Oligomeric double-stranded compounds can be two strands hybridized to form double-stranded compounds or a single strand with sufficient self complementarity to allow for hybridization and formation of a fully or partially double-stranded compound.

"Chimeric" oligomeric compounds or "chimeras," in the context of this invention, are single-or double-stranded oligomeric compounds, such as oligonucleotides, which contain two or more chemically distinct regions, each comprising at least one monomer unit, i.e., a nucleotide in the case of an oligonucleotide compound.

A "gapmer" is defined as an oligomeric compound, generally an oligonucleotide, having a 2'-deoxyoligonucleotide region flanked by non-deoxyoligonucleotide segments. The central region is referred to as the "gap." The flanking segments are referred to as "wings." If one of the wings has zero non-deoxyoligonucleotide monomers, a "hemimer" is described.

### NAFLD

The term "nonalcoholic fatty liver disease" (NAFLD) encompasses a disease spectrum ranging fro simple triglyceride accumulation in hepatocytes (hepatic steatosis) to hepatic steatosis with inflammation (steatohepatitis), fibrosis, and cirrhosis. Nonalcoholic steatohepatitis (NASH) occurs from progression of NAFLD beyond deposition of triglycerides. A second-hit capable of inducing necrosis, inflammation, and fibrosis is required for development of NASH. Candidates for the second-hit can be grouped into broad categories: factors causing an increase in oxidative stress and factors promoting expression of proinflammatory cytokines. It has been suggested that increased liver triglycerides lead to increased oxidative stress in hepatocytes of animals and humans, indicating a potential cause-and-effect relationship between hepatic triglyceride accumulation, oxidative stress, and the progression of hepatic steatosis to NASH (Browning and Horton, J. Clin. Invest., 2004, 114, 147-152). Hypertriglyceridemia and hyperfattyacidemia can cause triglyceride accumulation in peripheral tissues (Shimamura et al., Biochem. Biophys. Res. Commun., 2004,322,1080-1085*).* One embodiment of the present invention is a method of reducing lipids in the liver of an animal by administering a prophylactically or therapeutically effective amount of an oligomeric compound of the invention. Another embodiment of the present invention is a method of treating hepatic steatosis in an animal by administering a prophylactically or therapeutically effective amount of an oligomeric compound of the invention. In some embodiments, the steatosis is steatohepatitis. In some embodiments, the steatotis is NASH.

### Chemical Modifications

### Modified and Alternate Nucleobases

The oligomeric compounds of the invention also include variants in which a different base is present at one or more of the nucleotide positions in the compound. For example, if the first nucleotide is an adenosine, variants may be produced which contain thymidine, guanosine or cytidine at this position. This may be done at any of the positions of the oligomeric compound. These compounds are then tested using the methods described herein to determine their ability to reduce expression of GCCR mRNA.

Oligomeric compounds can also include nucleobase (often referred to in the art as heterocyclic base or simply as "base") modifications or substitutions. As used herein, "unmodified" or "natural" nucleobases include the purine bases adenine (A) and guanine (G), and the pyrimidine bases thymine (T), cytosine (C) and uracil (U). A "substitution" is the replacement of an unmodified or natural base with another unmodified or natural base. "Modified" nucleobases mean other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-CΞC-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine. Further modified nucleobases include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g. 9-(2-aminoethoxy)-H-pyrimido(5,4-b)(1,4)benzoxazin-2(3H)-one), carbazole cytidine (2H-pyrimido(4,5-b)indol-2-one), pyridoindole cytidine (H-pyrido(3',2':4,5)pyrrolo(2,3-d)pyrimidin-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in United States Patent No. 3,687,808, those disclosed in The Concise Encyclopedia Of Polymer Science And Engineering, pages 858-859, Kroschwitz, J.L, ed. John Wiley & Sons, 1990, those disclosed by Englisch et al., Angewandte Chemie, International Edition, 1991, 30, 613*,* and those disclosed by Sanghvi, Y.S., Chapter 15, Antisense Research and Applications, pages 289-302, Crooke, S.T. and Lebleu, B. , ed., CRC Press, 1993. Certain of these nucleobases are known to those skilled in the art as suitable for increasing the binding affinity of the compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2 °C and are presently suitable base substitutions, even more particularly when combined with 2'-O-methoxyethyl sugar modifications. It is understood in the art that modification of the base does not entail such chemical modifications as to produce substitutions in a nucleic acid sequence.

Representative United States patents that teach the preparation of certain of the above noted modified nucleobases as well as other modified nucleobases include, but are not limited to, the above noted U.S. 3,687,808, as well as U.S.: 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,645,985; 5,830,653; 5,763,588; 6,005,096; 5,681,941; and 5,750,692.

Oligomeric compounds of the present invention can also include polycyclic heterocyclic compounds in place of one or more of the naturally-occurring heterocyclic base moieties. A number of tricyclic heterocyclic compounds have been previously reported. These compounds are routinely used in antisense applications to increase the binding properties of the modified strand to a target strand. The most studied modifications are targeted to guanosines hence they have been termed G-clamps or cytidine analogs. Representative cytosine analogs that make 3 hydrogen bonds with a guanosine in a second strand include 1,3-diazaphenoxazine-2-one (Kurchavov, et al., Nucleosides and Nucleotides, 1997, 16, 1837-1846), 1,3-diazaphenothiazine-2-one , (Lin, K.-Y.; Jones, R. J.; Matteucci, M. J. Am. Chem. Soc. 1995, 117, 3873-3874) and 6,7,8,9-tetrafluoro-1,3-diazaphenoxazine-2-one (Wang, J.; Lin, K.-Y., Matteucci, M. Tetrahedron Lett. 1998, 39, 8385-8388). Incorporated into oligonucleotides these base modifications were shown to hybridize with complementary guanine and the latter was also shown to hybridize with adenine and to enhance helical thermal stability by extended stacking interactions (also see U.S. Pre-Grant Publications 20030207804 and 20030175906).

Further helix-stabilizing properties have been observed when a cytosine analog/substitute has an aminoethoxy moiety attached to the rigid 1,3-diazaphenoxazine-2-one scaffold (Lin, K.-Y.; Matteucci, M. J. Am. Chem. Soc. 1998, 120, 8531-8532). Binding studies demonstrated that a single incorporation could enhance the binding affinity of a model oligonucleotide to its complementary target DNA or RNA with a ΔTₘ of up to 18°C relative to 5-methyl cytosine (dC5^{me}), which is a high affinity enhancement for a single modification. On the other hand, the gain in helical stability does not compromise the specificity of the oligonucleotides.

Further tricyclic heterocyclic compounds and methods of using them that are amenable to use in the present invention are disclosed in United States Patents 6,028,183, and 6,007,992.

The enhanced binding affinity of the phenoxazine derivatives together with their uncompromised sequence specificity makes them valuable nucleobase analogs for the development of more potent antisense-based drugs. In fact, promising data have been derived from *in vitro* experiments demonstrating that heptanucleotides containing phenoxazine substitutions are capable to activate RNase H, enhance cellular uptake and exhibit an increased antisense activity (Lin, K-Y; Matteucci, M. J. Am. Chem. Soc. 1998, 120, 8531-8532). The activity enhancement was even more pronounced in case of G-clamp, as a single substitution was shown to significantly improve the *in vitro* potency of a 20mer 2'-deoxyphosphorothioate oligonucleotides (Flanagan, W. IVL; Wolf, J.J.; Olson, P.; Grant, D.; Lin, K.-Y.; Wagner, R. W.; Matteucci, M. Proc. Natl. Acad. Sci. USA, 1999, 96, 3513-3518).

Further modified polycyclic heterocyclic compounds useful as heterocyclic bases are disclosed in but not limited to, the above noted U.S. Patent 3,687,808, as well as U.S. Patents: 4,845,245; 5,130,302; 5,134,466; 5,175,273; 5,367,066; 5,432,272; 5,434,257; 5,457,187; 5,459,255; 5,484,908; 5,502,177; 5,525,711; 5,552,540; 5,587,469; 5,594,121, 5,596,091; 5,614,617; 5,645,985; 5,646,269; 5,750,692; 5,830,653; 5,763,588; 6,005,096; and 5,681,941, and U.S. Pre-Grant Publication 20030158403.

### Combinations

Compositions of the invention can contain two or more oligomeric compounds. In another related embodiment, compositions of the present invention can contain one or more antisense compounds, particularly oligonucleotides, targeted to a first nucleic acid and one or more additional antisense compounds targeted to a second nucleic acid target. Alternatively, compositions of the present invention can contain two or more antisense compounds targeted to different regions of the same nucleic acid target. Two or more combined compounds may be used together or sequentially.

### Combination therapy

The compounds of the invention may be used in combination therapies, wherein an additive effect is achieved by administering one or more compounds of the invention and one or more other suitable therapeutic/prophylactic compounds to treat a condition. Suitable therapeutic/prophylactic compound(s) include, but are not limited to, glucose-lowering agents, anti-obesity agents, and lipid lowering agents. Glucose lowering agents include, but are not limited to hormones, hormone mimetics, or incretin mimetics (e.g., insulin, including inhaled insulin, GLP-1 or GLP-1 analogs such as liraglutide, or exenatide), **DPP(IV)** inhibitors, a sulfonylurea (e.g., acetohexamide, chlorpropamide, tolbutamide, tolazamide, glimepiride, a glipizide, glyburide or a gliclazide), a biguanide (metformin), a meglitinide (e.g., nateglinide or repaglinide), a thiazolidinedione or other PPAR-gamma agonists (e.g., pioglitazone or rosiglitazone) an alpha-glucosidase inhibitor (e.g., acarbose or miglitol), or an antisense compound not targeted to GCGR. Also included are dual PPAR-agonists (e.g., muraglitazar, being developed by Bristol-Myers Squibb, or tesaglitazar, being developed by Astxa-Zeneca). Also included are other diabetes treatments in development (e.g. LAF237, being developed by Novartis; MK-0431, being developed by Merck; or rimonabant, being developed by Sanofi-Aventis). Anti-obesity agents include, but are not limited to, appetite suppressants (e.g. phentermine or Meridia™), fat absorption inhibitors such as orlistat (e.g. Xenical™), and modified forms of ciliary neurotrophic factor which inhibit hunger signals that stimulate appetite. Lipid lowering agents include, but are not limited to, bile salt sequestering resins (e.g., cholestyramine, colestipol, and colesevelam hydrochloride), HMGCoA-reductase inhibitors (e.g., lovastatin, pravastatin, atorvastatin, simvastatin, and fluvastatin), nicotinic acid, fibric acid derivatives (e.g., clofibrate, gemfibrozil, fenofibrate, bezafibrate, and ciprof'ibxate), probucol, neomycin, dextrothyroxine, plant-stanol esters, cholesterol absorption inhibitors (e.g., ezetimibe), CETP inhibitors (e.g. torcetrapib, and JTT-705) MTP inhibitors (eg, implitapide), inhibitors of bile acid transporters (apical sodium-dependent bile acid transporters), regulators of hepatic CYP7a, ACAT inhibitors (e.g. Avasimibe), estrogen replacement therapeutics (e.g., tamoxigen), synthetic HDL (e.g. ETC-216), antiinflammatories (e.g., glucocorticoids), or an antisense compound not targeted to GCGR. One or more of these drugs may be combined with one or more of the antisense inhibitors of GCGR to achieve an additive therapeutic effect.

### Oligomer Synthesis

Oligomerization of modified and unmodified nucleosides can be routinely performed according to literature procedures for DNA (Protocols for Oligonucleotides and Analogs, Ed. Agrawal (1993), Humana Press) and/or RNA (Scaringe, Methods (2001), 23, 206-217. Gait et al., Applications of Chemically synthesized RNA in RNA: Protein Interactions, Ed. Smith (1998), 1-36. Gallo et al., Tetrahedron (2001), 57, 5707-5713) and US Publication No. US2005-0164271, which is herein incorporated by reference.

Oligomeric compounds of the present invention can be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives.

### Oligomer Purification and Analysis

Methods of oligonucleotide purification and analysis are known to those skilled in the art. Analysis methods include capillary electrophoresis (CE) and electrospray-mass spectroscopy. Such synthesis and analysis methods can be performed in multi-well plates.

### Nonlinziting disclosure and incorporation by reference

While certain compounds, compositions and methods of the present invention have been described with specificity in accordance with certain embodiments, the examples herein serve only to illustrate the compounds of the invention and are not intended to limit the same. Each of the references, GENBANK® accession numbers, and the like recited in the present application is incorporated herein by reference in its entirety.

### Example 1

### Assaying Modulation of Expression

Modulation of GCCR expression can be assayed in a variety of ways known in the art. GCCR mRNA levels can be quantitated by, e.g., Northern blot analysis, competitive polymerase chain reaction (PCR), or real-time PCR. RNA analysis can be performed on total cellular RNA or poly(A)+ mRNA by methods known in the art. Methods of RNA isolation are taught in, for example, Ausubel, F.M. et al., Current Protocols in Molecular Biology, Volume 1, pp. 4.1.1-4.2.9 and 4.5.1-4.5.3, John Wiley & Sons, Inc., 1993**.**

Northern blot analysis is routine in the art and is taught in, for example, Ausubel, F.M. et al., Current Protocols in Molecular Biology, Volume 1, pp. 4.2.1-4.2.9, John Wiley & Sons, Inc., 1996. Real-time quantitative (PCR) can be conveniently accomplished using the commercially available ABI PRISM™ 7700 Sequence Detection System, available from PE-Applied Biosystems, Foster City, CA and used according to manufacturer's instructions.

Levels of proteins encoded by GCCR can be quantitated in a variety of ways well known in the art, such as immunoprecipitation, Western blot analysis (immunoblotting), ELISA or fluorescence-activated cell sorting (FACS). Antibodies directed to a protein encoded by GCCR can be identified and obtained from a variety of sources, such as the MSRS catalog of antibodies (Aerie Corporation, Birmingham, MI), or can be prepared via conventional antibody generation methods. Methods for preparation of polyclonal antisera are taught in, for example, Ausubel, F.M. et al., Current Protocols in Molecular Biology, Volume 2, pp. 11.12.1-11.12.9, John Wiley & Sons, Inc., 1997. Preparation of monoclonal antibodies is taught in, for example, Ausubel, F.M. et al., Current Protocols in Molecular Biology, Volume 2, pp. 11.4.1-11.11.5, John Wiley & Sons, Inc., 1997.

Immunoprecipitation methods are standard in the art and can be found at, for example, Ausubel, F.M. et al., Current Protocols in Molecular Biology, Volume 2, pp. 10.16.1-10.16.11, John Wiley & Sons, Inc., 1998. Western blot (immunoblot) analysis is standard in the art and can be found at, for example, Ausubel, F.M. et al., Current Protocols in Molecular Biology, Volume 2, pp. 10.8.1-10.8.21, John Wiley & Sons, Inc., 1997. Enzyme-linked immunosorbent assays (ELISA) are standard in the art and can be found at, for example, Ausubel, F.M. et al., Current Protocols in Molecular Biology, Volume 2, pp. 11.2.1-11.2.22, John Wiley & Sons, Inc., 1991.

The effect of oligomeric compounds of the present invention on target nucleic acid expression can be tested in any of a variety of cell types provided that the target nucleic acid is present at measurable levels. The effect of oligomeric compounds of the present invention on target nucleic acid expression can be routinely determined using, for example, PCR or Northern blot analysis. Cell lines are derived from both normal tissues and cell types and from cells associated with various disorders (e.g. hyperproliferative disorders). Cell lines derived from multiple tissues and species can be obtained from American Type Culture Collection (ATCC, Manassas, VA), the Japanese Cancer Research Resources Bank (Tokyo, Japan), or the Centre for Applied Microbiology and Research (Wiltshire, United Kingdom).

Primary cells, or those cells which are isolated from an animal and not subjected to continuous culture, can be prepared according to methods known in the art or obtained from various commercial suppliers. Additionally, primary cells include those obtained from donor human subj ects in a clinical setting (i.e. blood donors, surgical patients).

### Cell types

The effects of oligomeric compounds on target nucleic acid expression were tested in HepG2 cells and in primary rat hepatocytes.

### HepG2 cells:

The human hepatoblastoma cell line HepG2 was obtained from the American Type Culture Collection (Manassas, VA). HepG2 cells were routinely cultured in Eagle's MEM supplemented with 10% fetal bovine serum, 1 mM non-essential amino acids, and 1 mM sodium pyruvate (Invitrogen Life Technologies, Carlsbad, CA). Cells were routinely passaged by trypsinization and dilution when they reached approximately 90% confluence. Multiwell culture plates are prepared for cell culture by coating with a 1:100 dilution of type 1 rat tail collagen (BD Biosciences, Bedford, MA) in phosphate-buffered saline. The collagen-containing plates were incubated at 37°C for approximately 1 hour, after which the collagen was removed and the wells were washed twice with phosphate-buffered saline. Cells were seeded into 96-well plates (Falcon-Primaria #353872, BD Biosciences, Bedford, MA) at a density of approximately 8,000 cells/well for use in oligomeric compound transfection experiments.

### Primary rat hepatocytes:

Primary rat hepatocytes are prepared from Sprague-Dawley rats purchased from Charles River Labs (Wilmington, MA) and are routinely cultured in DMEM, high glucose (Invitrogen Life Technologies, Carlsbad, CA) supplemented with 10% fetal bovine serum (Invitrogen Life Technologies, Carlsbad, CA), 100 units per mL penicillin, and 100 µg/mL streptomycin (Invitrogen Life Technologies, Carlsbad, CA). Cells are seeded into 96-well plates (Falcon-Primaria #353872, BD Biosciences, Bedford, MA) at a density of approximately 4,000-6,000 cells/well treatment with the oligomeric compounds of the invention.

### Treatment with oligomeric compounds

When cells reached appropriate confluency, they were treated with oligonucleotide using a transfection method as described. Other suitable transfection reagents known in the art include, but are not limited to, LIPOFECTAMINE™, OLIGOFECTAMINE™, and FUGENE™. Other suitable transfection methods known in the art include, but are not limited to, electroporation.

### LIPOFECTIN™

When cells reach 65-75% confluency, they are treated with oligonucleotide. Oligonucleotide is mixed with LIPOFECTIN™ Invitrogen Life Technologies, Carlsbad, CA) in Opti-MEM™-1 reduced serum medium (Invitrogen Life Technologies, Carlsbad, CA) to achieve the desired concentration of oligonucleotide and a LIPOFECTIN™ concentration of 2.5 or 3 µg/mL per 100 nM oligonucleotide. This transfection mixture iss incubated at room temperature for approximately 0.5 hours. For cells grown in 96-well plates, wells are washed once with 100 µL OPTI-MEM™-1 and then treated with 130 µL of the transfection mixture. Cells grown in 24-well plates or other standard tissue culture plates are treated similarly, using appropriate volumes of medium and oligonucleotide. Cells are treated and data are obtained in duplicate or triplicate. After approximately 4-7 hours of treatment at 37°C, the medium containing the transfection mixture is replaced with fresh culture medium. Cells are harvested 16-24 hours after oligonucleotide treatment.

### CYTOFECTIN™

When cells reach 65-75% confluency, they are treated with oligonucleotide. Oligonucleotide is mixed with CYTOFECTIN™ (Gene Therapy Systems, San Diego, CA) in OPTI-MEM™-1 reduced serum medium (Invitrogen Life Technologies, Carlsbad, CA) to achieve the desired concentration of oligonucleotide and a CYTOFECTIN™ concentration of 2 or 4 µg/mL per 100 nM oligonucleotide. This transfection mixture is incubated at room temperature for approximately 0.5 hours. For cells grown in 96-well plates, wells are washed once with 100 µL OPTI-MEM™-1 and then treated with 130 µL of the transfection mixture. Cells grown in 24-well plates or other standard tissue culture plates are treated similarly, using appropriate volumes of medium and oligonucleotide. Cells are treated and data are obtained in duplicate or triplicate. After approximately 4-7 hours of treatment at 37°C, the medium containing the transfection mixture is replaced with fresh culture medium. Cells are harvested 16-24 hours after oligonucleotide treatment.

### Control oligonucleotides

Control oligonucleotides are used to determine the optimal oligomeric compound concentration for a particular cell line. Furthermore, when oligomeric compounds of the invention are tested in oligomeric compound screening experiments or phenotypic assays, control oligonucleotides are tested in parallel with compounds of the invention. In some-embodiments, the control oligonucleotides are used as negative control oligonucleotides, i.e., as a means for measuring the absence of an effect on gene expression or phenotype. In alternative embodiments, control oligonucleotides are used as positive control oligonucleotides, i.e., as oligonucleotides known to affect gene expression or phenotype. Control oligonucleotides are shown in Table 2. "Target Name" indicates the gene to which the oligonucleotide is targeted. "Species of Target" indicates species in which the oligonucleotide is perfectly complementary to the target mRNA. "Motif' is indicative of chemically distinct regions comprising the oligonucleotide. Certain compounds in Table 2 are chimeric oligonucleotides, composed of a central "gap" region consisting of 2'-deoxynucleotides, which is flanked on both sides (5' and 3') by "wings". The wings are composed of 2'-O-(2-methoxyethyl) nucleotides, also known as 2'-MOE nucleotides. The "motif" of each gapmer oligonucleotide is illustrated in Table 2 and indicates the number of nucleotides in each gap region and wing, for example, "5-10-5" indicates a gapmer having a 10-nucleotide gap region flanked by 5-nucleotide wings. ISIS 29848 is a mixture of randomized oligomeric compound; its sequence is shown in Table 2, where N can be A, T, C or G. The internucleoside (backbone) linkages are phosphorothioate throughout the oligonucleotides in Table 2. Unmodified cytosines are indicated by "°C" in the nucleotide sequence; all other cytosines are 5-methylcytosines.

**Table 2**

| **Control oligonucleotides for cell line testing, oligomeric compound screening and phenotypic assays** | | | | | |
|---|---|---|---|---|---|
| **ISIS#** | **Target Name** | **Species of Target** | **Sequence (5' to 3')** | **Motif** | **SEQ ID NO** |
| 113131 | CD86 | Human | CGTGTGTCTGTGCTAGTCCC | 5-10-5 | 4 |
| 289865 | forkhead box O1A (rhabdomyosarcoma ) | Human | GGCAACGTGAACAGGTCCAA | 5-10-5 | 5 |
| 25237 | integrin beta 3 | Human | GCCCATTGCTGGACATGC | z | 6 |
| 196103 | integrin beta 3 | Human | AGCCCATTGCTGGACATGCA | 5-10-5 | 7 |
| 148715 | Jagged 2 | Human; Mouse; Rat | TTGTCCCAGTCCCAGGCCTC | 5-10-5 | 8 |
| 18076 | Jun N-Terminal Kinase - 1 | Human | CTTTC"CGTTGGAUCUCCCTGGG | 5-9-6 | 9 |
| 18078 | Jun N-Terminal Kinase - 2 | Human | GTGCGᵘCGᵘCGAGᵘCᵘCᵘCGAAA TC | 5-9-6 | 10 |
| 183881 | kinesin-like 1 | Human | ATCCAAGTGCTACTGTAGTA | 5-10-5 | 11 |
| 29848 | none | none | NNNNNNNNNNNNNNNNNNNN N | 5-10-5 | 12 |
| 226844 | Notch (Drosophila) homolog 1 | Human; Mouse | GCCCTCCATGCTGGCACAGG | 5-10-5 | 13 |
| 105990 | Peroxisome proliferator-activated receptor gamma | Human | AGCAAAAGATCAATCCGTTA | 5-10-5 | 14 |
| 336806 | Raf kinase C | Human | TACAGAAGGCTGGGCCTTGA | 5-10-5 | 15 |

| **ISIS #** | **Target Name** | **Species of Target** | **Sequence (5' to 3')** | **Motif** | **SEQ ID NO** |
|---|---|---|---|---|---|
| 15770 | Raf kinase C | Mouse; Murine sarcoma virus; Rat | ATGCATT"CTG"C"C"C"C"CAAG GA | 5-10-5 | 16 |

The concentration of oligonucleotide used varies from cell line to cell line. To determine the optimal oligonucleotide concentration for a particular cell line, the cells are treated with a positive control oligonucleotide at a range of concentrations. Positive controls are shown in Table 2. For example, for human and non-human primate cells, the positive control oligonucleotide may be selected from ISIS 336806, or ISIS 18078. For mouse or rat cells the positive control oligonucleotide may be, for example, ISIS 15770. The concentration of positive control oligonucleotide that results in 80% reduction of the target mRNA, for example, rat Raf kinase C for ISIS 15770, is then utilized as the screening concentration for new oligonucleotides in subsequent experiments for that cell line. If 80% reduction is not achieved, the lowest concentration of positive control oligonucleotide that results in 60% reduction of the target mRNA is then utilized as the oligonucleotide screening concentration in subsequent experiments for that cell line. If 60% reduction is not achieved, that particular cell line is deemed as unsuitable for oligonucleotide transfection experiments. The concentrations of antisense oligonucleotides used herein are from 50 nM to 300 nM when the antisense oligonucleotide is transfected using a liposome reagent and 1 µM to 40 µM when the antisense oligonucleotide is transfected by electroporation.

### Example 2

### Real-time Quantitative PCR Analysis of GCCR mRNA Levels

Quantitation of GCCR mRNA levels was accomplished by real-time quantitative PCR using the ABI PRISM™ 7600, 7700, or 7900 Sequence Detection System (PE-Applied Biosystems, Foster City, CA) according to manufacturer's instructions.

Gene target quantities obtained by RT, real-time PCR were normalized using either the expression level of GAPDH, a gene whose expression is constant, or by quantifying total RNA using RiboGreen™ (Molecular Probes, Inc. Eugene, OR). Total RNA was quantified using RiboGreen™ RNA quantification reagent (Molecular Probes, Inc. Eugene, OR). 170 µL of RiboGreen™ working reagent (RiboGreen™ reagent diluted 1:350 in 10mM Tris-HCl, 1 mM EDTA, pH 7.5) was pipetted into a 96-well plate containing 30 µL purified cellular RNA. The plate was read in a CytoFluor 4000 (PE Applied Biosystems) with excitation at 485nm and emission at 530nm.

GAPDH expression was quantified by RT, real-time PCR, either simultaneously with the quantification of the target or separately. For measurement simultaneous with measurement of target levels, primer-probe sets specific to the target gene being measured were evaluated for their ability to be "multiplexed" with a GAPDH amplification reaction prior to quantitative PCR analysis. Multiplexing refers to the detection of multiple DNA species, in this case the target and endogenous GAPDH control, in a single tube, which requires that the primer-probe set for GAPDH does not interfere with amplification of the target.

Probes and primers for use in real-time PCR were designed to hybridize to target-specific sequences. Methods of primer and probe design are known in the art. Design of primers and probes for use in real-time PCR can be carried out using commercially available software, for example Primer Express®, PE Applied Biosystems, Foster City, CA. The primers and probes and the target nucleic acid sequences to which they hybridize are presented in Table 4. The target-specific PCR probes have FAM covalently linked to the 5' end and TAMRA or MGB covalently linked to the 3' end, where FAM is the fluorescent dye and TAMRA or MGB is the quencher dye.

After isolation, the RNA is subjected to sequential reverse transcriptase (RT) reaction and real-time PCR, both of which are performed in the same well. RT and PCR reagents were obtained from Invitrogen Life Technologies (Carlsbad, CA). RT, real-time PCR was carried out in the same by adding 20 µL PCR cocktail (2.5x PCR buffer minus MgCl₂, 6.6 mM MgCl₂, 375 µM each of dATP, dCTP, dCTP and dGTP, 375 nM each of forward primer and reverse primer, 125 nM of probe, 4 Units RNAse inhibitor, 1.25 Units PLATINUM® Taq, 5 Units MuLV reverse transcriptase, and 2.5x ROX dye) to 96-well plates containing 30 µL total RNA solution (20-200 ng). The RT reaction was carried out by incubation for 30 minutes at 48°C. Following a 10 minute incubation at 95°C to activate the PLATINUM® Taq, 40 cycles of a two-step PCR protocol were carried out: 95°C for 15 seconds (denaturation) followed by 60°C for 1.5 minutes (annealing/extension).

Compounds of the invention were evaluated for their effect on human target mRNA levels by quantitative real-time PCR as described in other examples herein, using a primer-probe set designed to hybridize to human GCCR. For example:
Forward primer: TTGACATTTTGCAGGATTTGGA (incorporated herein as SEQ ID NO: 17)
Reverse primer: CCAAGGACTCTCATTCGTCTCTTT (incorporated herein as SEQ ID NO: 18)

And the PCR probe:
FAM-TTTCTTCTGGGTCCCC-MGB (incorporated herein as SEQ ID NO: 19), where FAM is the fluorescent dye and MGB is a non-fluorescent quencher dye.

Compounds of the invention were evaluated for their effect on rat target mRNA levels by quantitative real-time PCR as described in other examples herein, using a primer-probe set designed to hybridize to rat GCCR. For example:
Forward primer: AAACAATAGTTCCTGCAGCATTACC (incorporated herein as SEQ ID NO: 20)
Reverse primer: CATAGAACACCTCGGGTTCAATC (incorporated herein as SEQ ID NO: 21)

And the PCR probe:
FAM- ACCCCTACCTTGGTGTCACTGCT-TAMRA (incorporated herein as SEQ ID NO: 22), where
FAM is the fluorescent dye and TAMRA is the quencher dye.

Compounds of the invention can be evaluated for their effect on mouse target mRNA levels by quantitative real-time PCR as described in other examples herein, using a primer-probe set designed to hybridize to mouse GCCR. For example:
Forward primer: GACATCTTGCAGGATTTGGAGTT (incorporated herein as SEQ ID NO: 23)
Reverse primer: AACAGGTCTGACCTCCAAGGACT (incorporated herein as SEQ ID NO: 24)

And the PCR probe:
FAM- CGGGTCCCCAGGTAAAGAGACAAACGA-TAMRA (incorporated herein as SEQ ID NO: 25),
where FAM is the fluorescent dye and TAMRA is the quencher dye.

### Example 3

### Antisense inhibition of human GCCR expression by 5-10-5 gapmers

A series of oligomeric compounds was designed to target different regions of human GCCR, using published sequences cited in Table 1. The compounds are shown in Table 3. All compounds in Table 3 are chimeric oligonucleotides ("gapmers") 20 nucleotides in length, composed of a central "gap" region consisting of 10 2'-deoxynucleotides, which is flanked on both sides (5' and 3') by five-nucleotide "wings". The wings are composed of 2'-O-(2-methoxyethyl) nucleotides, also known as 2'-MOE nucleotides. The internucleoside (backbone) linkages are phosphorothioate throughout the oligonucleotide. All cytidine residues are 5-methylcytidines. Shown in Table 3 is the sequence of the oligonucleotide, and the target site which is the first (5' most) position on the target sequence to which the compound binds. The compounds were analyzed for their effect on gene target mRNA levels by _ quantitative real-time PCR as described in other examples herein, using a primer-probe set designed to hybridize to human GCCR.

Data are averages from three experiments in which HepG2 cells were treated with 50 nM of the disclosed oligomeric compounds using LIPOFECTIN™. A reduction in expression is expressed as percent inhibition in Table 3. If present, "N.D." indicates "not determined". The target regions to which these oligomeric compounds are inhibitory are herein referred to as "validated target segments."

**Table 3**

| **Inhibition of human GCCR mRNA levels by 5-10-5 gapmers** | | | | | |
|---|---|---|---|---|---|
| **ISIS No of 5-10-5** | **Target SEQ ID NO** | **Target Site** | **Sequence** | **% Inhib w/5-10-5** | **SEQ ID NO** |
| 361132 | 1 | 394 | TCTGTCTCTCCCATATACAG | 65 | 26 |
| 361133 | 1 | 398 | TGTTTCTGTCTCTCCCATAT | 56 | 27 |
| 361134 | 1 | 402 | CTTTTGTTTCTGTCTCTCCC | 60 | 28 |
| 361135 | 1 | 406 | ATCACTTTTGTTTCTGTCTC | 80 | 29 |
| 180272 | 1 | 497 | GTTTGCAATGCTTTCTTCCA | 74 | 30 |
| 345188 | 1 | 501 | TGAGGTTTGCAATGCTTTCT | 71 | 31 |
| 361136 | 1 | 505 | CTATTGAGGTTTGCAATGCT | 10 | 32 |
| 361137 | 1 | 509 | CGACCTATTGAGGTTTGCAA | 80 | 33 |
| 180274 | 1 | 514 | CTGGTCGACCTATTGAGGTT | 68 | 34 |
| 180275 | 1 | 672 | CTGTGGTATACAATTTCACA | 44 | 35 |
| 180276 | 1 | 679 | CTTTGGTCTGTGGTATACAA | 78 | 36 |
| 345198 | 1 | 689 | GTCAAAGGTGCTTTGGTCTG | 79 | 37 |
| 180279 | 1 | 877 | GGTTTAGTGTCCGGTAAAAT | 60 | 38 |
| 361138 | 1 | 954 | CTTTTTCTGTTTTCACTTGG | 70 | 39 |
| 180280 | 1 | 1000 | TTCTCTTGCTTAATTACCCC | 77 | 40 |
| 345218 | 1 | 1004 | CAGTTTCTCTTGCTTAATTA | 67 | 41 |
| 180281 | 1 | 1007 | GCCCAGTTTCTCTTGCTTAA | 74 | 42 |
| 361139 | 1 | 1058 | TTTATTACCAATTATATTTG | 0 | 43 |
| 361140 | 1 | 1062 | ACATTTTATTACCAATTATA | 35 | 44 |
| 361141 | 1 | 1066 | GCAGACATTTTATTACCAAT | 78 | 45 |
| 361142 | 1 | 1070 | AATGGCAGACATTTTATTAC | 40 | 46 |
| 361143 | 1 | 1074 | CAGAAATGGCAGACATTTTA | 63 | 47 |
| 361144 | 1 | 1078 | TGAACAGAAATGGCAGACAT | 61 | 48 |
| 180283 | 1 | 1081 | CCATGAACAGAAATGGCAGA | 69 | 49 |
| 361145 | 1 | 1085 | CACACCATGAACAGAAATGG | 30 | 50 |
| 361146 | 1 | 1089 | TACTCACACCATGAACAGAA | 60 | 51 |
| 361147 | 1 | 1093 | GAGGTACTCACACCATGAAC: | 71 | 52 |
| 361148 | 1 | 1097 | TCCAGAGGTACTCACACCAT | 75 | 53 |
| 361149 | 1 | 1101 | GTCCTCCAGAGGTACTCACA | 69 | 54 |
| 361150 | 1 | 1105 | ATCTGTCCTCCAGAGGTACT | 53 | 55 |
| 361151 | 1 | 1109 | GTACATCTGTCCTCCAGAGG | 75 | 56 |
| 361152 | 1 | 1113 | AGTGGTACATCTGTCCTCCA | 62 | 57 |
| 361153 | 1 | 1117 | JTCATAGTGGTACATCTGTCC | 52 | 58 |
| 361154 | 1 | 1121 | CATGTCATAGTGGTACATCT | 57 | 59 |
| 361155 | 1 | 1125 | TATTCATGTCATAGTGGTAC | 41 | 60 |
| 361156 | 1 | 1129 | GCTGTATTCATGTCATAGTG | 67 | 61 |
| 361157 | 1 | 1133 | GGATGCTGTATTCATGTCAT | 67 | 62 |
| 361158 | 1 | 1137 | AAAGGGATGCTGTATTCATG | 45 | 63 |
| 180288 | 1 | 1141 | TGAGAAAGGGATGCTGTATT | 62 | 64 |
| 180289 | 1 | 1181 | TGGTGGAATGACATTAAAAA | 54 | 65 |
| 361159 | 1 | 1185 | GAATTGGTGGAATGACATTA | 24 | 66 |
| 361160 | 1 | 1324 | GAGCTTACATCTGGTCTCAT | 59 | 67 |
| 361161 | 1 | 1328 | AGGAGAGCTTACATCTGGTC | 65 | 68 |
| 361162 | 1 | 1332 | ATGGAGGAGAGCTTACATCT | 18 | 69 |
| 361163 | 1 | 1336 | CTGGATGGAGGAGAGCTTAC | 50 | 70 |
| 361164 | 1 | 1339 | GAGCTGGATGGAGGAGAGCT | 49 | 71 |
| 361165 | 1 | 1468 | TGTCCTTCCACTGCTCTTTT | 61 | 72 |
| 361166 | 1 | 1472 | GTGCTGTCCTTCCACTGCTC | 65 | 73 |
| 361167 | 1 | 1476 | AATTGTGCTGTCCTTCCACT | 62 | 74 |
| 361168 | 1 | 1480 | AGGTAATTGTGCTGTCCTTC | 52 | 75 |
| 361169 | 1 | 1543 | CGGCATGCTGGGCAGTTTTT | 78 | 76 |
| 361170 | 1 | 1547 | ATAGCGGCATGCTGGGCAGT | 58 | 77 |
| 361171 | 1 | 1549 | CGATAGCGGCATGCTGGGCA | 65 | 78 |
| 361172 | 1 | 1570 | ATTCCAGCCTGAAGACATTT | 24 | 79 |
| 361173 | 1 | 1574 | GTTCATTCCAGCCTGAAGAC | 52 | 80 |
| 361174 | 1 | 1597 | TTCTTTGTTTTTCGAGCTTC | 62 | 81 |
| 361175 | 1 | 1601 | TTTTTTCTTTGTTTTTCGAG | 48 | 82 |
| 180297 | 1 | 1680 | CAGGAACTATTGTTTTGTTA | 33 | 83 |
| 361176 | 1 | 1682 | TGCAGGAACTATTGTTTTGT | 46 | 84 |
| 361177 | 1 | 1765 | GAGCTATCATATCCTGCATA | 71 | 85 |
| 361178 | 1 | 1769 | AACAGAGCTATCATATCCTG | 51 | 86 |
| 361179 | 1 | 1773 | CTGGAACAGAGCTATCATAT | 67 | 87 |
| 361180 | 1 | 1840 | TTCACTGCTGCAATCACTTG | 52 | 88 |
| 361181 | 1 | 1844 | CCATTTCACTGCTGCAATCA | 55 | 89 |
| 361182 | 1 | 1848 | TTGCCCATTTCACTGCTGCA | 70 | 90 |
| 361183 | 1 | 1999 | ATAATCAGATCAGGAGCAAA | 36 | 91 |
| 361184 | 1 | 2003 | ATTAATAATCAGATCAGGAG | 10 | 92 |
| 361185 | 1 | 2007 | GCTCATTAATA.ATCAGATCA | 43 | 93 |
| 361186 | 1 | 2011 | CTCTGCTCATTAATAATCAG | 0 | 94 |
| 180302 | 1 | 2015 | CATTCTCTGCTCATTAATA.A | 23 | 95 |
| 180304 | 1 | 2053 | AGCATGTGTTTACATTGGTC | 73 | 96 |
| 361187 | 1 | 2119 | AAGGTTTTCATACAGAGATA | 38 | 97 |
| 361188 | 1 | 2123 | CAGTAAGGTTTTCATACAGA | 22 | 98 |
| 361189 | 1 | 2127 | GAAGCAGTAAGGTTTTCATA | 46 | 99 |
| 180307 | 1 | 2131 | GAGAGAAGCAGTAAGGTTTT | 32 | 100 |
| 361190 | 1 | 2212 | GCTTTTCCTAGCTCTTTGAT | 74 | 101 |
| 361191 | 1 | 2215 | ATGGCTTTTCCTAGCTCTTT | 68 | 102 |
| 361192 | 1 | 2347 | ATGGTCTTATCCAAAAATGT | 63 | 103 |
| 361193 | 1 | 2351 | ACTCATGGTCTTATCCAAAA | 66 | 104 |
| 361194 | 1 | 2355 | CAATACTCATGGTCTTATCC | 54 | 105 |
| 361195 | 1 | 2359 | AATTCAATACTCATGGTCTT | 69 | 106 |
| 361196 | 1 | 2383 | ATGATTTCAGCTAACATCTC | 1 | 107 |
| 180311 | 1 | 2386 | GTGATGATTTCAGCTAACAT | 59 | 108 |
| 361197 | 1 | 2407 | GAATATTTTGGTATCTGATT | 59 | 109 |
| 361198 | 1 | 2411 | ATTTGAATATTTTGGTATCT | 20 | 110 |
| 361199 | 1 | 2415 | TTCCATTTGAATATTTTGGT | 65 | 111 |
| 361200 | 1 | 2419 | ATATTTCCATTTGAATAT'TT | 51 | 112 |
| -361202 | -1 | - 2425 - | TTTTTGATATTTCCATTTGA | 20 | 113 |

The 5-10-5 gapmer oligonucleotides shown in Table 3 which reduced GCCR expression by at least 30% are preferred. The target segments to which these preferred sequences are complementary are herein referred to as "preferred target segments" and are therefore preferred for targeting by compounds of the present invention. Another aspect of the present invention is an antisense compound targeted to GCCR comprising an 8-nucleobase portion of SEQ ID NOs: 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94,95,96,97,98,99,100,101,102,103,104,105,106,107,108,109,110,111,112,or 113 wherein said compound specifically hybridizes with and reduces expression of GCCR. In one embodiment the antisense compound is an antisense oligonucleotide, 20 nucleobases in length characterized by a 10-deoxynucleotide region flanked on its 5' and 3' ends with five 2'-O-(2-methoxyethyl) nucleotides. In one embodiment, all of the internucleoside linkages are phosphorothioate linkages. In one embodiment, all of the cytosines are 5-methylcytosines.

### Example 4

### Antisense inhibition of human GCCR expression by gap-widened oligonucleotides

In accordance with the present invention, gap-widened oligonucleotides having the same sequences as the compounds described in Table 4 were also tested. All compounds in Table 4 are chimeric oligonucleotides ("gapmers") 20 nucleotides in length, composed of a central "gap" region consisting of 16 2'-deoxynucleotides, which is flanked on both sides (5' and 3') by two-nucleotide "wings". The wings are composed of 2'-O-(2-methoxyethyl) nucleotides, also known as 2'-MOE nucleotides. The internucleoside (backbone) linkages are phosphorothioate throughout the oligonucleotide. All cytidine residues are 5-methylcytidines. Shown in Table 4 is the sequence of the oligonucleotide, and the target site which is the first (5' most) position on the target sequence to which the compound binds. The 2-16-2 motif compounds were analyzed for their effect on gene target mRNA levels by quantitative real-time PCR as described herein.

Data are averages from three experiments in which HepG2 cells were treated with 50 nM of the disclosed oligomeric compounds using LIPOFECTIN™. A reduction in expression is expressed as percent inhibition in Table 4. If present, "N.D." indicates "not determined". The target regions to which these oligomeric compounds are inhibitory are herein referred to as "validated target segments." "

**Table 4**

| **Inhibition of human GCCR mRNA levels by 2-16-2 gapmers** | | | | | |
|---|---|---|---|---|---|
| **ISIS No of 2-16-2** | **Target SEQ IDNO** | **Target Site** | **Sequence** | **% Inhib w/2-16-2** | **SEQ ID NO** |
| 372350 | 1 | 394 | TCTGTCTCTCCCATATACAG | 69 | 26 |
| 372376 | 1 | 398 | TGTTTCTGTCTCTCCCATAT | 72 | 27 |
| 372331 | 1 | 402 | CTTTTGTTTCTGTCTCTCCC | 67 | 28 |
| 372341 | 1 | 406 | ATCACTTTTGTTTCTGTCTG | 63 | 29 |
| 352983 | 1 | -497 | GTTTGCAATGCTTTCTTCCA | 64 | 30 |
| 372365 | 1 | 501 | TGAGGTTTGCAATGCTTTCT | 69 | 31 |
| 372387 | 1 | 505 | CTATTGAGGTTTGCAATGCT | 70 | 32 |
| 372316 | 1 | 509 | CGACCTATTGAGGTTTGCAA | 73 | 33 |
| 372310 | 1 | 514 | CTGGTCGACCTATTGAGGTT | 70 | 34 |
| 372315 | 1 | 672 | CTGTGGTATACAATTTCACA | 35 | 35 |
| 372326 | 1 | 679 | CTTTGGTCTGTGGTATACAA | 54 | 36 |
| 372339 | 1 | 689 | GTCAAAGGTGCTTTGGTCTG | 81 | 37 |
| 372322 | 1 | 877 | GGTTTAGTGTCCGGTAAAAT | 78 | 38 |
| 372361 | 1 | 954 | CTTTTTCTGTTTTCACTTGG | 70 | 39 |
| 372308 | 1 | 1000 | TTCTCTTGCTTAATTACCCC | 84 | 40 |
| 372304 | 1 | 1004 | CAGTTTCTCTTGCTTAATTA | 66 | 41 |
| 352984 | 1 | 1007 | GCCCAGTTTCTCTTGCTTAA | 80 | 42 |
| 372372 | 1 | 1058 | TTTATTACCAATTATATTTG | 0 | 43 |
| 372327 | 1 | 1062 | ACATTTTATTACCAA.TTATA | 11 | 44 |
| 372311 | 1 | 1066 | GCAGACATTTTATTACCAAT | 65 | 45 |
| 372352 | 1 | 1070 | AATGGCAGACATTTTATTAC | 54 | 46 |
| 372337 | 1 | 1074 | CAGAAATGGCAGACATTTTA | 36 | 47 |
| 372323 | 1 | 1078 | TGAACAGAAATGGCAGACAT | 73 | 48 |
| 372347 | 1 | 1081 | CCATGAACAGAAATGGCAGA | 86 | 49 |
| 372383 | 1 | 1085 | CACACCATGAACAGAAATGG | 73 | 50 |
| 372348 | 1 | 1089 | TACTCACACCATGAACAGAA | 82 | 51 |
| 372363 | 1 | 1093 | GAGGTACTCACACCATGAAC | 47 | 52 |
| 372334 | 1 | 1097 | TCCAGAGGTACTCACACCAT | 82 | 53 |
| 372359 | 1 | 1101 | GTCCTCCAGAGGTACTCACA | 69 | 54 |
| 372344 | 1 | 1105 | ATCTGTCCTCCAGAGGTACT | 72 | 55 |
| 372307 | 1 | 1109 | GTACATCTGTCCTCCAGAGG | 74 | 56 |
| 372370 | 1 | 1113 . | AGTGGTACATCTGTCCTCCA | 69 | 57 |
| 372374 | 1 | 1117 | TCATAGTGGTACATCTGTCC | 0 | 58 |
| 372355 | 1 | 1121 | CATGTCATAGTGGTACATCT | 65 | 59 |
| 372385 | 1 | 1125 | TATTCATGTCATAGTGGTAC | 18 | 60 |
| 372319 | 1 | 1129 | GCTGTATTCATGTCATAGTG | 23 | 61 |
| 372366 | 1 | 1133 | GGATGCTGTATTCATGTCAT | 37 | 62 |
| 372330 | 1 | 1137 | AAAGGGATGCTGTATTCATG | 80 | 63 |
| 372333 | 1 | 1141 | TGAGAAAGGGATGCTGTATT | 68 | 64 |
| 372358 | 1 | 1181 | TGGTGGAATGACATTAAAAA | 67 | 65 |
| 372381 | 1 | 1185 | GAATTGGTGGAATGACATTA | 30 | 66 |
| 372377 | 1 | 1324 | GAGCTTACATCTGGTCTCAT | 45 | 67 |
| 372309 | 1 | 1328 | AGGAGAGCTTACATCTGGTC | 63 | 68 |
| 372388 | 1 | 1332 | ATGGAGGAGAGCTTACATCT | 55 | 69 |
| 372321 | 1 | 1336 | CTGGATGGAGGAGAGCTTAC | 51 | 70 |
| 372312 | 1 | 1339 | GAGCTGGATGGAGGAGAGCT | 60 | 71 |
| 372324 | 1 | 1468 | TGTCCTTCCACTGCTCTTTT | 73 | 72 |
| 372332 | 1 | 1472 | GTGCTGTCCTTCCACTGCTC | 81 | 73 |
| 372335 | 1 | 1476 | AATTGTGCTGTCCTTCCACT | 42 | 74 |
| 372342 | 1 | 1480 | AGGTAATTGTGCTGTCCTTC | 100 | 75 |
| 372345 | 1 | 1543 | CGGCATGCTGGGCAGTTTTT | 82 | 76 |
| 372356 | 1 | 1547 | ATAGCGGCATGCTGGGCAGT | 73 | 77 |
| 372305 | 1 | 1549 | CGATAGCGGCATGCTGGGCA | 80 | 78 |
| 372367 | 1 | 1570 | ATTCCAGCCTGAAGACATTT | 78 | 79 |
| 372353 | 1 | 1574 | GTTCATTCCAGCCTGAAGAC | 70 | 80 |
| 372364 | 1 | 1597 | TTCTTTGTTTTTCGAGCTTC | 47 | 81 |
| 372340 - | 1 | 1601 | TTTTTTCTTTGTTTTTCGAG | 100 | 82 |
| 372369 | 1 | 1680 | CAGGAACTATTGTTTTGTTA | 56 | 83 |
| 372378 | 1 | 1682 | TGCAGGAACTATTGTTTTGT | 41 | 84 |
| 372317 | 1 | 1765 | GAGCTATCATATCCTGCATA | 84 | 85 |
| 372351 | 1 | 1769 | AACAGAGCTATCATATCCTG | 69 | 86 |
| 372389 | 1 | 1773 | CTGGAACAGAGCTATCATAT | 76 | 87 |
| 372362 | 1 | 1840 | TTCACTGCTGCAATCACTTG | 64 | 88 |
| 372328 | 1 | 1844 | CCATTTCACTGCTGCAATCA | 81 | 89 |
| 372338 | 1 | 1848 | TTGCCCATTTCACTGCTGCA | 82 | 90 |
| 372349 | 1 | 1999 | ATAATCAGATCAGGAGCAAA | 10 | 91 |
| 372373 | 1 | 2003 | ATTAATAATCAGATCAGGAG | 30 | 92 |
| 372360 | 1 | 2007 | GCTCATTAATAATCAGATCA | 27 | 93 |
| 372384 | 1 | 2011 | CTCTGCTCATTAATAATCAG | 100 | 94 |
| 372380 | 1 | 2015 | CATTCTCTGCTCATTAATAA | 2 | 95 |
| 372320 | 1 | 2053 | AGCATGTGTTTACATTGGTC | 75 | 96 |
| 372371 | 1 | 2119 | AAGGTTTTCATACAGAGATA | 37 | 97 |
| 372382 | 1 | 2123 | CAGTAAGGTTTTCATACAGA | 44 | 98 |
| 372306 | 1 | 2127 | GAAGCAGTAAGGTTTTCATA | 48 | 99 |
| 372343 | 1 | 2131 | GAGAGAAGCAGTAAGGTTTT | 46 | 100 |
| 372313 | 1 | 2212 | GCTTTTCCTAGCTCTTTGAT | 66 | 101 |
| 372325 | 1 | 2215 | ATGGCTTTTCCTAGCTCTTT | 69 | 102 |
| 372336 | 1 | 2347 | ATGGTCTTATCCAAAAATGT | 65 | 103 |
| 372318 | 1 | 2351 | ACTCATGGTCTTATCCAAAA | 70 | 104 |
| 372375 | 1 | 2355 | CAATACTCATGGTCTTATCC | 85 | 105 |
| 372346 | 1 | 2359 | AATTCAATACTCATGGTCTT | 47 | 106 |
| 372386 | 1 | 2383 | ATGATTTCAGCTAACATCTC | 74 | 107 |
| 372354 | 1 | 2386 | GTGATGATTTCAGCTAACAT | 66 | 108 |
| 372357 | 1 | 2407 | GAATATTTTGGTATGTGATT | 13 | 109 |
| 372368 | 1 | 2411 | ATTTGAATATTTTGGTATCT | 0 | 110 |
| 372379 | 1 | 2415 | TTCCATTTGAATATTTTGGT | 44 | 111 |
| 372390 | 1 | 2419 | ATATTTCCATTTGAATATTT | 0 | 112' |
| 372329 | 1 | 2425 | TTTTTGATATTTCCATTTGA | 0 | 113 |

The 2-16-2 oligonucleotides shown in Table 4 which reduced GCCR expression by at least 30% are preferred. The target segments to which these preferred sequences are complementary are herein referred to as "preferred target segments" and are therefore preferred for targeting by compounds of the present invention,

Another aspect of the present invention is an antisense compound targeted to GCCR comprising an 8-nucleobase portion of SEQ ID NOs: 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 9, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, or 113 wherein said compound specifically hybridizes with and reduces expression of GCCR. In one embodiment the antisense compound is an antisense oligonucleotide, 20 nucleobases in length characterized by a 16-deoxynucleotide region flanked on its 5' and 3' ends with two 2'-O-(2-methoxyethyl) nucleotides. In one embodiment, all of the internucleoside linkages are phosphorothioate linkages. In one embodiment, all of the cytosines are 5-methylcytosines.

### Example 5

### Cross-species oligonucleotides targeting GCCR

Some oligonucleotides described in the previous example are complementary across species and are therefore expected to reduce expression of glucocorticoid receptor across species. Shown in Table 5 is the sequence of such cross-species oligonucleotides, and the ISIS numbers of the 5-10-5 motif version and the 2-16-2 motif version of the oligonucleotide. Also indicated for each sequence is the target site which is the first (5' most) position on the human target sequence (NM_000176.1, incorporated herein as SEQ ID NO: 1) to which the compound binds. The complementarity for human, cynomolgus monkey, rat, and mouse GCCR mRNA is indicated ("yes" means perfect complementarity and "I mm" means one mismatch from perfect complementarity).

**Table 5**

| **Cross-species oligonucleotides targeted to GCCR** | | | | | | | | |
|---|---|---|---|---|---|---|---|---|
| **ISIS # of 5-10-5 gapmer** | **ISIS # of 2-16-2 gapmer** | **SEQ ID NO** | **Sequence** | **Pos'n on SEQ ID NO: 1** | **Perfect complement to:** | | | |
| | | | | | **Humann** | **Monkey** | **Rat** | **Mouse** |
| 361137 | 372316 | 33 | cgacctattgaggtttgca a | 509 | yes | yes | yes | yes |
| 180276 | 372326 | 36 | ctttggtctgtggtatacaa | 679 | yes | 1 mm | 1mm | yes |
| 345198 | 372339 | 37 | gtcaaaggtgctttggtct g | 689 | yes | yes | yes | yes |
| 180304 | 372320 | 96 | agcatgtgtttacattggtc | 2053 | yes | yes | yes | yes |
| 180275 | 372315 | 35 | ctgtggtatacaatttcaca | 672 | yes | 1 mm | 1mm | yes |
| 361141 | 372311 | 45 | gcagacattttattaccaat | 1066 | yes | yes | yes | 1 mm |
| 180281 | 352984 | 42 | gcccagtttctcttgcttaa | 1007 | yes | yes | yes | yes |
| 361151 | 372307 | 56 | gtacatctgtcctccagag g | 1109 | yes | yes | yes | yes |
| 180274 | 372310 | 34 | ctggtcgacctattgaggt t | 514 | yes | yes | yes | yes |
| 361156 | 372319 | 61 | gctgtattcatgtcatagtg | 1129 | yes | yes | yes | yes |

### Example 6

### Antisense inhibition of human and rat GCCR mRNA levels-dose-response studies with 5-10-5 gapmers

In a further embodiment of the present invention, eleven oligonucleotides were selected for additional dose-response studies. Primary rat hepatocytes were treated with 5, 10, 25, 50, 100 or 200 nM of ISIS 180274, ISIS 180275, ISIS 180276, ISIS 180281, ISIS 180304, ISIS 361137, ISIS 361141, ISIS 361151, ISIS 361156, ISIS 345198, ISIS 361137 or the negative control oligonucleotide ISIS 141923 (CCTTCCCTGAAGGTTCCTCC, incorporated herein as SEQ ID NO: 114), and mRNA levels were measured as described in other examples herein. ISIS 141923 is a 5-10-5 gapmer comprising a ten deoxynucleotide gap flanked by 2'-MOE wings and a phosphorothioate backbone. All cytosines are 5-methylcytosines. Untreated cells served as the control to which the data were normalized.

Results of these studies are shown in Table 6. Target mRNA levels were measured by real-time PCR as described herein. Data are averages from three experiments and are expressed as percent inhibition relative to untreated control.

**Table 6**

| **Dose-dependent inhibition of GCCR expression in rat primary hepatocytes** | | | | | | | |
|---|---|---|---|---|---|---|---|
| **ISIS #** | **SEQ ID NO** | **% Inhibition** | | | | | |
| | | **Dose of Oligonucleotide (nM)** | | | | | |
| | | **5** | **10** | **25** | **50** | **100** | **200** |
| 180274 | 34 | 16 | 33 | 29 | 65 | 84 | 89 |
| 180275 | 35 | 0 | 13 | 56 | 84 | 84 | 90 |
| 180276 | 36 | 23 | 43 | 43 | 68 | 89 | 93 |
| 180281 | 42 | 0 | 20 | 33 | 75 | 86 | 87 |
| 180304 | 96 | 42 | 51 | 47 | 75 | 86 | 91 |
| 361137 | 33 | 40 | 30 | 48 | 81 | 83 | 89 |
| 361141 | 45 | 36 | 61 | 48 | 77 | 87 | 92 |
| 361151 | 56 | 10 | 28 | 42 | 77 | 90 | 94 |
| 361156 | 61 | 22 | 47 | 46 | 66 | 84 | 92 |
| 345198 | 37 | 0 | 35 | 53 | 81 | 77 | 85 |
| 361158 | 63 | 34 | 50 | 47 | 79 | 91 | 93 |
| 141923 | 114 | 0 | 10 | 18 | 43 | 0 | 12 |

In a further embodiment of the present invention, the same oligonucleotides were tested in the human HepG2 cell line for their ability to reduce GCCR mRNA expression at the indicated doses.

Untreated cells served as the control to which the data were normalized.

Results of these studies are shown in Table 7. Target mRNA levels were measured by real-time PCR as described herein. Data are averages from three experiments and are expressed as percent inhibition relative to untreated control.

**Table 7**

| **Dose-dependent inhibition of GCCR expression in HepG2 cells** | | | | | | | |
|---|---|---|---|---|---|---|---|
| **ISIS #** | **SEQ ID NO** | **% Inhibition** | | | | | |
| | | **Dose of Oligonucleotide (nM)** | | | | | |
| | | **1** | **10** | **25** | **50** | **100** | **200** |
| 180274 | 34 | 0 | 31 | 54 | 66 | 77 | 83 |
| 180275 | 35 | 13 | 54 | 75 | 86 | 93 | 94 |
| 180276 | 36 | 26 | 77 | 87 | 92 | 94 | 98 |
| 180281 | 42 | 3 | 46 | 68 | 80 | 90 | 84 |
| 180304 | 96 | 0 | 64 | 90 | 90 | 92 | 91 |
| 361137 | 33 | 18 | 71 | 84 | 91 | 92 | 86 |
| 361141 | 45 | 1 | 49 | 81 | 85 | 73 | 78 |
| 361151 | 56 | 22 | 42 | 71 | 82 | 89 | 91 |
| 361156 | 61 | 7 | 75 | 75 | 79 | 80 | 82 |
| 345198 | 37 | 17 | 71 | 79 | 86 | 80 | 82 |
| 361158 | 63 | 11 | 35 | 78 | 80 | 82 | 77 |
| 141923 | 114 | 15 | 12 | 20 | 12 | 14 | 3 |

As shown in Table 6 and Table 7, antisense oligonucleotides targeting GCCR are effective at reducing both human and rat target mRNA levels in a dose-dependent manner.

### Example 7

### Antisense inhibition of rat GCCR mRNA levels-in vivo dose-response studies with 5-10-5 gapmers

Five of the 5-10-5 gapmer motif oligonucleotides (ISIS 180281, ISIS 361137, ISIS 345198, ISIS 180304, and ISIS 361141) were evaluated at various doses in rats for their ability to reduce GCCR mRNA levels in liver. Eight week-old Sprague Dawley rats were divided into treatment groups which received doses of 50, 25 or 12.5 mg/kg of one the indicated oligonucleotides via injection. Each treatment group was comprised of four animals, and was dosed twice weekly for 3 weeks. Animals injected with saline alone served as a control group. The animals were evaluated weekly for standard blood parameters (ALT/AST, cholesterol, triglycerides, and glucose). Animals were sacrificed at the end of the study and liver tissue was collected and analyzed for target reduction using real-time PCR analysis methods described herein. Results are shown in Tables 8a and 8b (separate experiments) as the percentage reduction in GCCR mRNA measured after treatment with the indicated doses of the indicated oligonucleotides.

**Table 8a**

| **In vivo rat screen- GCCR antisense oligonucleotides** | | | |
|---|---|---|---|
| **Compound** | **% Reduction in GCCR mRNA in rat liver (compared to saline-treated controls)** | | |
| | **50 mg/kg** | **25 mg/kg** | **12.5 mg/kg** |
| ISIS 180281 | 68 | 65 | 48 |
| ISIS 180304 | 52 | 34 | 0 |
| ISIS 345198 | 63 | 58 | 52 |

**Table 8b**

| **In vivo rat screen- GCCR antisense oligonucleotides** | | | |
|---|---|---|---|
| **Compound** | **% Reduction in GCCR mRNA in rat liver (compared to saline-treated controls)** | | |
| | **50 mg/kg** | **25 mg/kg** | **12.5 mg/kg** |
| ISIS180281 | 62 | 62 | 59 |
| ISIS 361137 | 59 | 47 | 32 |
| ISIS 361141 | 61 | 49 | 22 |

The data in Tables 8a and 8b show that antisense oligonucleotides targeted to GCCR are effective at reducing expression in vivo in a dose-dependent manner. ISIS 345198 (GTCAAAGGTGCTTTGGTCTG; SEQ ID NO: 37) was chosen for further evaluation in structure-activity experiments focusing on gap optimization. This compound is perfectly complementary to mouse, rat, human, monkey, rabbit and guinea pig glucocorticoid receptor RNA.

### Example 8

### Antisense inhibition of GCCR mRNA levels in vivo—gap optimization study

A series of oligomeric compounds were designed to target GCCR with varying sizes of the deoxynucleotide gap and 2'-MOE wings. Each of the oligonucleotides tested has the same nucleobase sequence (GTCAAAGGTGCTTTGGTCTG, incorporated herein as SEQ NO: 37) and therefore targets the same segment of SEQ ID NO: 1 (nucleobases 689 to 709). As shown in Example 5*,* this oligonucleotide is also perfectly complementary to rat GCCR.

The compounds are shown in Table 9. Plain text indicates a deoxynucleotide, and nucleobases designated with **bold, underlined** text are 2'-O-(2-methoxyethyl) nucleotides. Internucleoside linkages are phosphorothioate throughout, and all cytosines are 5-methylcytosines. Indicated in Table 9 is the "motif" of each compound indicative of chemically distinct regions comprising the oligonucleotide.

**Table 9**

| **Antisense compounds targeting rat GCCR** | | | |
|---|---|---|---|
| **ISIS Number** | **Chemistry** | **SEQ ID NO:** | **Motif** |
| 345198 | **GTCAA**AGGTGCTTTG**GTCTG** | 37 | 5-10-5 gapmer |
| 372339 | **GT**CAAAGGTGCTTTGGTC**TG** | 37 | 2-16-2 gapmer |
| 377130 | **GTC**AAAGGTGCTTTGGT**CTG** | 37 | 3-14-3 gapmer |
| 377131 | **GTCA**AAGGTGCTTTGGTCTG | 37 | 4-12-4 gapmer |

Nine-week old Sprague-Dawley male rats were treated twice weekly for three weeks with doses of 50, 25, 12.5, and 6.25 mg/kg of the oligonucleotides presented in Table 9. Animals injected with saline alone served as controls. Each treatment group was comprised of four animals and animals were monitored weekly for plasma transamunases, lipids, glucose levels and body weight gain. As expected for normal animals, no substantial alterations in glucose were observed. Baseline (prior to the start of treatment) plasma cholesterol (CHOL) and triglyceride (TRIG) levels and levels measured at week 3 are shown in Table 10 in mg/dL as the average for each treatment group.

**Table 10**

| **Effect of oligonucleotides targeted to GCCR on plasma lipids levels in normal rats** | | | | |
|---|---|---|---|---|
| **Treatment** | **Baseline TRIG (mg/dL)** | **Week 3 TRIG (mg/dL)** | **Baseline CHOL (mg/dL)** | **Week 3 CHOL (mg/dL)** |
| Saline | 78 | 70 | 77 | 62 |
| 345198, 50 mg/kg | 50 | 23 | 66 | 35 |
| 345198, 25 mg/kg | 99 | 34 | 69 | 39 |
| 345198, 12.5 mg/kg | 71 | 52 | 64 | 42 |
| 345I98, 6.25 mg/lcg | 139 | 99 | 78 | 58 |
| 372339, 50 mg/kg | 93 | 29 | 75 | 54 |
| 372339, 25mg/kg | 86 | 33 | 70 | 40 |
| 372339, 12.5 mg/kg | 104 | 71 | 69 | 49 |
| 372339, 6.25 mg/kg | 103 | 102 | 71 | 56 |
| 377130, 50 mg/kg | 91 | 21 | 65 | 41 |
| 377130, 25 mg/kg | 82 | 32 | 75 | 41 |
| 377130, 12.5 mg/kg | 84 | 68 | 72 | 47 |
| 377130, 6.25 mg/kg | 76 | 67 | 70 | 52 |
| 377131, 50 mg/kg | 96 | 28 | 85 | 48 |
| 377131, 25 mg/kg | 83 | 25 | 75 | 42 |
| 377131, 12.5 mg/kg | 64 | 49 | 79 | 44 |
| 377131, 6.25 mg/kg | 119 | 110 | 75 | 60 |

As shown in Table 10, treatment with antisense oligonucleotides targeted to GCCR caused dose-dependent decreases in cholesterol and triglyceride levels. Therefore, one embodiment of the present invention is a method of decreasing blood lipid levels in an animal comprising administering to said animal a gap-widened oligonucleotide. In a preferred embodiment, the gap-widened oligonucleotide has the sequence of SEQ ID NO: 37. In other preferred embodiments, the gap-widened oligonucleotide is ISIS 372339, ISIS 37713Q, or ISIS 377131.

At the end of the study, animals were sacrificed, organ weights were measured, and tissues were collected for determination of target reduction and oligonucleotide concentration.

White adipose tissue was analyzed for target reduction using real-time PCR analysis methods described herein. Results are shown in Tables 11a, 11b, and 11c (separate experiments) as the percentage reduction in GCCR mRNA measured after treatment with the indicated doses of the indicated oligonucleotides. Tissues from animals treated with each gap-widened oligonucleotide were assayed for target reduction alongside tissues from animals treated with the 5-10-S motif oligonucleotide for

**Table 12a**

| **In vivo reduction of GCCR levels in white adipose tissue with 2-16-2 oligonucleotides** | | | | |
|---|---|---|---|---|
| **Treatment group** | **% Inhibition** | | | |
| | **Dose of oligonucleotide (mg/kg)** | | | |
| | **50** | **25** | **12.5** | **6.25** |
| **ISIS 345198** | 56 | 26 | 17 | 7 |
| **ISIS 372339** | 34 | 0 | 8 | 8 |

**Table 11b**

| **In vivo reduction of GCCR levels in white adipose tissue with 3-14-3 oligonucleotides** | | | | |
|---|---|---|---|---|
| **Treatment group** | **% Inhibition** | | | |
| | **Dose of oligonucleotide (mg/kg**) | | | |
| | **50** | **25** | **12.5** | **6.25** |
| ISIS 345198 | 59 | 49 | 27 | 22 |
| ISIS 377130 | 54 | 37 | 21 | 18 |

**Table 11c**

| **In vivo reduction of GCCR levels in white adipose tissue with 4-12-4 oligonucleotides** | | | | |
|---|---|---|---|---|
| **Treatment group** | **% Inhibition** | | | |
| | **Dose of oligonucleotide (mg/kg)** | | | |
| | **50** | **25** | **12.5** | **6.25** |
| ISIS 345198 | 56 | 23 | 21 | 7 |
| ISIS 377131 | 55 | 23 | 15 | 0 |

Liver tissue was also analyzed for target reduction using real-time PCR analysis methods described herein. Results are shown in Tables 12a, 12b, and 12c (separate experiments) as the percentage reduction in GCCR mRNA measured after treatment with the indicated doses of the indicated oligonucleotides. Tissues from animals treated with each gap-widened oligonucleotide were assayed for target reduction alongside tissues from animals treated with the 5-10-5 motif oligonucleotide for comparison.

**Table 12a**

| **In vivo reduction of GCCR levels in liver with 2-16-2 oligonucleotides** | | | | |
|---|---|---|---|---|
| **Treatment group** | **% Inhibition** | | | |
| | **Dose of oligonucleotide (mg/kg)** | | | |
| | **50** | **25** | **12.5** | **6.25** |
| ISIS 345198 | 78 | 77 | 65 | 51 |
| ISIS 372339 | 83 | 77 | 56 | 44 |

**Table 12b**

| **In vivo reduction of GCCR levels in liver with 3-14-3 oligonucleotides** | | | | |
|---|---|---|---|---|
| **Treatment group** | **% Inhibition** | | | |
| | **Dose of oligonucleotide (mg/kg)** | | | |
| | **50** | **25** | **12.5** | **1 6.25** |
| ISIS 345198 | 78 | 80 | 67 | 54 |
| ISIS 377130. | 87 | 78 | 68 | 43 |

**Table 12c**

| **In vivo reduction of GCCR levels in liver with 4-12-4 oligonucleotides** | | | | |
|---|---|---|---|---|
| **Treatment group** | **% Inhibition** | | | |
| | **Dose of oligonucleotides (mg/kg)** | | | |
| | **50** | **25** | **12.5** | **6.25** |
| ISIS 345198 | 76 | 75 | 58 | 49 |
| ISIS 377131 | 82 | 64 | 60 | 61 |

As shown in Tables 11a, 11b, and 11c all of the gap-widened oligonucleotides tested were effective at reducing GCCR levels in a dose-dependent manner in vivo. In addition, the gap-widened oligonucleotides show a trend toward greater potency than the 5-10-5 gapmer in the liver.

In addition, to determine effects of altering the gap size on pharmacokinetics, oligonucleotide concentration in kidney and liver were determined. Methods to determine oligonucleotide concentration in tissues are known in the art (Geary et al., Anal Biochem, 1999, 274, 241-248). Total oligonucleotide is the sum of all oligonucleotides metabolites detected in the tissue. Shown in Table 12 are the total concentration and the concentration of full length oligonucleotide (in µg/g) in the liver of animals treated with the indicated oligonucleotide at the indicated concentration.

**Table 12**

| **GCCR oligonucleotide concentration in rat liver** | | | | |
|---|---|---|---|---|
| **Treatment** | **Motif** | **Dose** | **Liver Total oligo** | **Liver Full-length** |
| **ISIS 345198** | **5-10-5** | 25 mg/kg | 507 | 408 |
| | | 12.5mg/kg | 318 | 224 |
| **ISIS 372339** | **2-16-2** | 25 mg/kg | 450 | 306 |
| | | 12.5 mg/kg | 311 | 183 |
| **ISIS 377130** | **3-14-3** | 25 mg/kg | 575 | 315 |
| | | 12.5 mg/kg | 350 | 212 |
| **ISIS 377131** | **4-12-4** | 25 mg/kg | 584 | 424 |
| | | 12.5 mg/kg | 354 | 265 |

As shown in Table 12, the levels of full-length oligonucleotide in the liver are comparable or reduced tor ISIS 372339 and ISIS 377130 as compared to ISIS 345198. Coupled with the target reduction as shown in Table 11, these data show that the enhanced potency of the gap-widened compounds is not due to enhanced accumulation of the compound in the liver. Thus, preferred oligonucleotides of the present invention include gap-widened oligonucleotides that show enhanced or comparable potency with regard to target reduction to the corresponding 5-1o-5 gapmer without enhanced accumulation of the compound in a target tissue. In some embodiments, the target tissue is adipose and in some embodiments, the target tissue is liver.

The invention also provides the following numbered embodiments:
1. An antisense oligonucleotide 20 nucleobases in length targeted to a nucleic acid molecule encoding GCCR and comprising at least an 8-nucleobase portion of SEQ ID NO: 34, 33, 35, 36, 37, 42, 45, 56, 61, 63, or 96, wherein the oligonucleotide comprises a deoxynucleotide region 12, 13, 14, 15, 16, 17, or 18 nucleobases in length which is flanked on its 5' and 3' ends with 1 to 4 2'-O-(2-methoxyethyl) nucleotides and wherein the oligonucleotide specifically hybridizes to and reduces expression of GCCR.
2. The antisense oligonucleotide of embodiment 1 wherein the number of nucleotides flanking the deoxynucleotide region on the 5' and 3' ends is the same.
3. The antisense oligonucleotide of embodiment 1 wherein the number of nucleotides flanking the deoxynucleotide region on the 5' and 3' ends is not the same.
4. The antisense oligonucleotide of embodiment 1 wherein at least one internucleoside linkage is a phosphorothioate linkage.
5. The antisense oligonucleotide of embodiment 1 wherein at least one cytosine is a 5-methylcytosine.
6. The antisense oligonucleotide of embodiment 1 having the nucleobase sequence of SEQ ID NO: 37.
7. The antisense oligonucleotide of embodiment 6 characterized by a 16-deoxynucleotide region flanked on its 5' and 3' ends with two 2'-O-(2-methoxyethyl) nucleotides.
8. The antisense oligonucleotide of embodiment 7 wherein at least one internucleoside linkage is a phosphorothioate linkage.
9. The antisense oligonucleotide of embodiment 7 wherein at least one cytosine is a 5-methylcytosine.
10. The antisense oligonucleotide of embodiment 6 characterized by a 14-deoxynucleotide region flanked on its 5' and 3' ends with three 2'-O-(2-methoxyethyl) nucleotides.
11. The antisense oligonucleotide of embodiment 10 wherein at least one internucleoside linkage is a phosphorothioate linkage.
12. The antisense oligonucleotide of embodiment 10 wherein at least one cytosine is a 5-methylcytosine.
13. The antisense oligonucleotide of embodiment 6 characterized by a 12-deoxynucleotide region flanked on its 5' and 3' ends with four 2'-O-(2-methoxyethyl) nucleotides.
14. The antisense oligonucleotide of embodiment 13 wherein at least one internucleoside linkage is a phosphorothioate linkage.
15. The antisense oligonucleotide of embodiment 13 wherein at least one cytosine is a 5-methylcytosine.
16. The antisense oligonucleotide of embodiment 6 characterized by an 18-deoxynucleotide region flanked on its 5' and 3' ends with one 2'-O-(2-methoxyethyl) nucleotides.
17. The antisense oligonucleotide of embodiment 16 wherein at least one internucleoside linkage is a phosphorothioate linkage.
18. The antisense oligonucleotide of embodiment 16 wherein at least one cytosine is a 5-methylcytosine.
19. The antisense oligonucleotide of embodiment 6 characterized by a 17-deoxynucleotide region flanked on its 5' and 3' ends with one or two 2'-O-(2-methoxyethyl) nucleotides.
20. The antisense oligonucleotide of embodiment 19 wherein at least one internucleoside linkage is a phosphorothioate linkage.
21. The antisense oligonucleotide of embodiment 19 wherein at least one cytosine is a 5-methylcytosine.
22. The antisense oligonucleotide of embodiment 1 having the nucleobase sequence of SEQ ID NO: 33.
23. The antisense oligonucleotide of embodiment 22 characterized by a 16-deoxynucleotide region flanked on its 5' and 3' ends with two 2'-O-(2-methoxyethyl) nucleotides.
24. The antisense oligonucleotide of embodiment 23 wherein at least one internucleoside linkage is a phosphorothioate linkage.
25. The antisense oligonucleotide of embodiment 23 wherein at least one cytosine is a 5-methylcytosine.
26. The antisense oligonucleotide of embodiment 22 characterized by a 14-deoxynucleotide region flanked on its 5' and 3' ends with three 2'-O-(2-methoxyethyl) nucleotides.
27. The antisense oligonucleotide of embodiment 26 wherein at least one internucleoside linkage is a phosphorothioate linkage.
28. The antisense oligonucleotide of embodiment 26 wherein at least one cytosine is a 5-methylcytosine.
29. The antisense oligonucleotide of embodiment 22 characterized by a 12-deoxynucleotide region flanked on its 5' and 3' ends with four 2'-O-(2-methoxyethyl) nucleotides.
30. The antisense oligonucleotide of embodiment 29 wherein at least one internucleoside linkage is a phosphorothioate linkage.
31. The antisense oligonucleotide of embodiment 29 wherein at least one cytosine is a 5-methylcytosine.
32. The antisense oligonucleotide of embodiment 22 characterized by an 18-deoxynucleotide region flanked on its 5' and 3' ends with one 2'-O-(2-methoxyethyl) nucleotides.
33. The antisense oligonucleotide of embodiment 32 wherein at least one internucleoside linkage is a phosphorothioate linkage.
34. The antisense oligonucleotide of embodiment 32 wherein at least one cytosine is a 5-methylcytosine.
35. The antisense oligonucleotide of embodiment 22 characterized by a 17-deoxynucleotide region flanked on its 5' and 3' ends with one or two 2'-O-(2-methoxyethyl) nucleotides.
36. The antisense oligonucleotide of embodiment 35 wherein at least one internucleoside linkage is a phosphorothioate linkage.
37. The antisense oligonucleotide of embodiment 35 wherein at least one cytosine is a 5-methylcytosine.
38. The antisense oligonucleotide of embodiment 1 having the nucleobase sequence of SEQ ID NO: 45.
39. The antisense oligonucleotide of embodiment 38 characterized by a 16-deoxynucleotide region flanked on its 5' and 3' ends with two 2'-O-(2-methoxyethyl) nucleotides.
40. The antisense oligonucleotide of embodiment 39 wherein at least one internucleoside linkage is a phosphorothioate linkage.
41. The antisense oligonucleotide of embodiment 39 wherein at least one cytosine is a 5-methylcytosine.
42. The antisense oligonucleotide of embodiment 38 characterized by a 14-deoxynucleotide region flanked on its 5' and 3' ends with three 2'-O-(2-methoxyethyl) nucleotides.
43. The antisense oligonucleotide of embodiment 42 wherein at least one internucleoside linkage is a phosphorothioate linkage.
44. The antisense oligonucleotide of embodiment 42 wherein at least one cytosine is a 5-methylcytosine.
45. The antisense oligonucleotide of embodiment 38 characterized by a 12-deoxynucleotide region flanked on its 5' and 3' ends with four 2'-O-(2-methoxyethyl) nucleotides.
46. The antisense oligonucleotide of embodiment 45 wherein at least one internucleoside linkage is a phosphorothioate linkage.
47. The antisense oligonucleotide of embodiment 45 wherein at least one cytosine is a 5-methylcytosine.
48. The antisense oligonucleotide of embodiment 38 characterized by an 18-deoxynucleotide region flanked on its 5' and 3' ends with one 2'-O-(2-methoxyethyl) nucleotides.
49. The antisense oligonucleotide of embodiment 48 wherein at least one internucleoside linkage is a phosphorothioate linkage.
50. The antisense oligonucleotide of embodiment 48 wherein at least one cytosine is a 5-methylcytosine.
51. The antisense oligonucleotide of embodiment 38 characterized by a 17-deoxynucleotide region flanked on its 5' and 3' ends with one or two 2'-O-(2-methoxyethyl) nucleotides.
52. The antisense oligonucleotide of embodiment 51 wherein at least one internucleoside linkage is a phosphorothioate linkage.
53. The antisense oligonucleotide of embodiment 51 wherein at least one cytosine is a 5-methylcytosine.
54. A pharmaceutical composition comprising the antisense oligonucleotide of embodiment 1 and optionally a pharmaceutically acceptable carrier, diluent enhancer or excipient.
55. A method of reducing expression of glucocorticoid receptor in a cell or tissue comprising contacting said cell or tissue with the pharmaceutical composition of embodiment 54.
56. The method of embodiment 55 wherein the tissue is fat or liver tissue.
57. A method of treating a disease or condition mediated by glucocorticoid expression in an animal comprising contacting said animal with an effective amount of the pharmaceutical composition of embodiment 54.
58. The method of embodiment 57 wherein the disease or condition is diabetes, obesity, metabolic syndrome X, hyperglycemia, or hyperlipidemia.
59. The method of embodiment 57 wherein the disease is Type 2 diabetes.
60. The method of embodiment 57 wherein the disease is hyperlipidemia associated with elevated blood cholesterol or elevated blood triglyceride levels.
61. The method of embodiment 57 wherein the condition is liver steatosis.
62. The method of embodiment 61 wherein the steatosis is steatohepatitis .
63. The method of embodiment 61 wherein the steatosis is non-alcoholic steatohepatitis
64. A method of decreasing blood glucose levels in an animal comprising administering to said animal a therapeutically effective amount of the pharmaceutical composition of embodiment 54.
65. The method of embodiment 64 wherein the animal is a human.
66. The method of embodiment 64 wherein blood glucose levels are fasting blood glucose levels.
67. A method of decreasing blood lipid levels in an animal comprising administering to said animal a therapeutically effective amount of the pharmaceutical composition of embodiment 54.
68. The method of embodiment 67 wherein blood lipid levels are blood cholesterol levels.
69. The method of embodiment 67 wherein blood lipid levels are blood triglyceride levels.
70. A method of decreasing liver triglyceride levels in an animal comprising administering to said animal a therapeutically effective amount of the pharmaceutical composition of embodiment 54.
71. A method of reducing body fat mass in an animal comprising administering to said animal a therapeutically effective amount of the pharmaceutical composition of embodiment 54.
72. A method of reducing improving insulin sensitivity in an animal comprising administering to said animal a therapeutically effective amount of the pharmaceutical composition of embodiment 54.
73. A method of inhibiting hepatic glucose output in an animal comprising administering to said animal a therapeutically effective amount of the pharmaceutical composition of embodiment 54.
74. A method of delaying or preventing the onset of an increase in blood lipid or blood glucose levels in an animal comprising administering to said animal a therapeutically effective amount of the pharmaceutical composition of embodiment 54.
75. A method of treating an animal having a metabolic disease or condition comprising administering to said animal a compound of embodiment 1 in combination with an antidiabetic agent selected from the group comprising PPAR agonists including PPAR-gamma, dual-PPAR or pan-PPAR agonists, dipeptidyl peptidase (IV) inhibitors, GLP-1 analogs, insulin and insulin analogues, insulin secretogogues, SGLT2 inihibitors, human amylin analogs including pramlintide, glucokinase activators, biguanides and alpha - glucosidase inhibitors to achieve an additive therapeutic effect.

## Claims

1. An antisense oligonucleotide 13 to 26 nucleobases in length targeted to a nucleic acid molecule encoding glucocorticoid receptor (GCCR) wherein the oligonucleotide comprises a first region, a second region, and a third region, wherein said first region comprises at least 11 deoxynucleotides, wherein said second and third regions comprise at least one 2'-O-(2-methoxyethyl) nucleotide, wherein said second and third regions flank the first region on the 5' and 3' ends of said first region, and wherein said oligonucleotide specifically hybridizes to a nucleic acid molecule encoding GCCR and reduces the expression of GCCR.

2. The oligonucleotide of claim 1, wherein the deoxynucleotide region is 11, 12, 13, 14, 15, 16, 17 or 18 deoxynucleotides in length.

3. The oligonucleotide of claim 1 or claim 2, wherein the deoxynucleotide region is flanked on each of its 5' and 3' ends by 1, 2, 3 or 4 2'-O-(2-methoxyethyl) nucleotides.

4. The oligonucleotide of any of claims 1-3, wherein the flanking regions are symmetrical.

5. The oligonucleotide of any of claims 1-3, wherein the flanking regions are non-symmetrical.

6. The oligonucleotide of any of claims 1-5, wherein the oligonucleotide contains at least one modified internucleoside linkage.

7. The oligonucleotide of claim 6, wherein at least one internucleoside linkage is a phosphorothioate linkage.

8. The oligonucleotide of any of claims 1-7, wherein the oligonucleotide contains at least one modified nucleobase.

9. The oligonucleotide of claim 8, wherein at least one cytosine is a 5-methylcytosine.

10. A pharmaceutical composition comprising an antisense oligonucleotide of any of claims 1-9 and a pharmaceutically acceptable carrier, diluent, enhancer or excipient.

11. A method of reducing the expression of GCCR in cells or tissues, comprising contacting said cells or tissues *in vitro* with an oligonucleotide of any of claims 1-9 or a pharmaceutical composition of claim 10.

12. An antisense oligonucleotide of any of claims 1-9 or a pharmaceutical composition of claim 10, for use in:
(a) reducing the expression of GCCR in cells, tissues or animals;
(b) decreasing blood glucose levels, blood triglyceride levels, or blood cholesterol levels in an animal;
(c) increasing insulin sensitivity, decreasing liver triglyceride levels, or inhibiting hepatic glucose output in an animal;
(d) reducing body fat mass in an animal; or
(e) preventing or delaying the onset of elevated blood glucose or blood lipid levels in an animal.

13. The oligonucleotide or composition of claim 12, for use in reducing the expression of GCCR in liver tissue or adipose tissue.

14. An antisense oligonucleotide of any of claims 1-9 or a pharmaceutical composition of claim 10, for use in treating a metabolic disease or condition.

15. The oligonucleotide or composition of claim 14, for use in:
(i) treating diabetes, obesity, metabolic syndrome X, hyperglycemia, hyperlipidemia, or insulin resistance; or
(ii) treating liver steatosis, such as steatohepatitis or non-alcoholic steatohepatitis.


SEQUENCE LISTING

<110> Isis Pharmaceuticals, Inc.

<120> MODULATION OF GLUCOCORTICOID RECEPTOR EXPRESSION

<130> BIOL0065EP.D1

<150> 60/718,685
<151> 2005-09-19

<160> 114

<170> FastSEQ for Windows Version 4.0

<210> 1
<211> 4788
<212> DNA
<213> H. sapiens

<400> 1
tttttagaaa aaaaaaatat atttccctcc tgctccttct gcgttcacaa gctaagttgt 60
ttatctcggc tgcggcggga actgcggacg gtggcgggcg agcggctcct ctgccagagt 120
tgatattcac tgatggactc caaagaatca ttaactcctg gtagagaaga aaaccccagc 180
agtgtgcttg ctcaggagag gggagatgtg atggacttct ataaaaccct aagaggagga 240
gctactgtga aggtttctgc gtcttcaccc tcactggctg tcgcttctca atcagactcc 300
aagcagcgaa gacttttggt tgattttcca aaaggctcag taagcaatgc gcagcagcca 360
gatctgtcca aagcagtttc actctcaatg ggactgtata tgggagagac agaaacaaaa 420
gtgatgggaa atgacctggg attcccacag cagggccaaa tcagcctttc ctcgggggaa 480
acagacttaa agcttttgga agaaagcatt gcaaacctca ataggtcgac cagtgttcca 540
gagaacccca agagttcagc atccactgct gtgtctgctg cccccacaga gaaggagttt 600
ccaaaaactc actctgatgt atcttcagaa cagcaacatt tgaagggcca gactggcacc 660
aacggtggca atgtgaaatt gtataccaca gaccaaagca cctttgacat tttgcaggat 720
ttggagtttt cttctgggtc cccaggtaaa gagacgaatg agagtccttg gagatcagac 780
ctgttgatag atgaaaactg tttgctttct cctctggcgg gagaagacga ttcattcctt 840
ttggaaggaa actcgaatga ggactgcaag cctctcattt taccggacac taaacccaaa 900
attaaggata atggagatct ggttttgtca agccccagta atgtaacact gccccaagtg 960
aaaacagaaa aagaagattt catcgaactc tgcacccctg gggtaattaa gcaagagaaa 1020
ctgggcacag tttactgtca ggcaagcttt cctggagcaa atataattgg taataaaatg 1080
tctgccattt ctgttcatgg tgtgagtacc tctggaggac agatgtacca ctatgacatg 1140
aatacagcat ccctttctca acagcaggat cagaagccta tttttaatgt cattccacca 1200
attcccgttg gttccgaaaa ttggaatagg tgccaaggat ctggagatga caacttgact 1260
tctctgggga ctctgaactt ccctggtcga acagtttttt ctaatggcta ttcaagcccc 1320
agcatgagac cagatgtaag ctctcctcca tccagctcct caacagcaac aacaggacca 1380
cctcccaaac tctgcctggt gtgctctgat gaagcttcag gatgtcatta tggagtctta 1440
acttgtggaa gctgtaaagt tttcttcaaa agagcagtgg aaggacagca caattaccta 1500
tgtgctggaa ggaatgattg catcatcgat aaaattcgaa gaaaaaactg cccagcatgc 1560
cgctatcgaa aatgtcttca ggctggaatg aacctggaag ctcgaaaaac aaagaaaaaa 1620
ataaaaggaa ttcagcaggc cactacagga gtctcacaag aaacctctga aaatcctggt 1680
aacaaaacaa tagttcctgc aacgttacca caactcaccc ctaccctggt gtcactgttg 1740
gaggttattg aacctgaagt gttatatgca ggatatgata gctctgttcc agactcaact 1800
tggaggatca tgactacgct caacatgtta ggagggcggc aagtgattgc agcagtgaaa 1860
tgggcaaagg caataccagg tttcaggaac ttacacctgg atgaccaaat gaccctactg 1920
cagtactcct ggatgtttct tatggcattt gctctggggt ggagatcata tagacaatca 1980
agtgcaaacc tgctgtgttt tgctcctgat ctgattatta atgagcagag aatgactcta 2040
ccctgcatgt acgaccaatg taaacacatg ctgtatgttt cctctgagtt acacaggctt 2100
caggtatctt atgaagagta tctctgtatg aaaaccttac tgcttctctc ttcagttcct 2160
aaggacggtc tgaagagcca agagctattt gatgaaatta gaatgaccta catcaaagag 2220
ctaggaaaag ccattgtcaa gagggaagga aactccagcc agaactggca gcggttttat 2280
caactgacaa aactcttgga ttctatgcat gaagtggttg aaaatctcct taactattgc 2340
ttccaaacat ttttggataa gaccatgagt attgaattcc ccgagatgtt agctgaaatc 2400
atcaccaatc agataccaaa atattcaaat ggaaatatca aaaaacttct gtttcatcaa 2460
aagtgactgc cttaataaga atggttgcct taaagaaagt cgaattaata gcttttattg 2520
tataaactat cagtttgtcc tgtagaggtt ttgttgtttt attttttatt gttttcatct 2580
gttgttttgt tttaaatacg cactacatgt ggtttataga gggccaagac ttggcaacag 2640
aagcagttga gtcgtcatca cttttcagtg atgggagagt agatggtgaa atttattagt 2700
taatatatcc cagaaattag aaaccttaat atgtggacgt aatctccaca gtcaaagaag 2760

gatggcacct aaaccaccag tgcccaaagt ctgtgtgatg aactttctct tcatactttt 2820
tttcacagtt ggctggatga aattttctag actttctgtt ggtgtatccc ccccctgtat 2880
agttaggata gcatttttga tttatgcatg gaaacctgaa aaaaagttta caagtgtata 2940
tcagaaaagg gaagttgtgc cttttatagc tattactgtc tggttttaac aatttccttt 3000
atatttagtg aactacgctt gctcattttt tcttacataa ttttttattc aagttattgt 3060
acagctgttt aagatgggca gctagttcgt agctttccca aataaactct aaacattaat 3120
caatcatctg tgtgaaaatg ggttggtgct tctaacctga tggcacttag ctatcagaag 3180
accacaaaaa ttgactcaaa tctccagtat tcttgtcaaa aaaaaaaaaa aaaaagctca 3240
tattttgtat atatctgctt cagtggagaa ttatataggt tgtgcaaatt aacagtccta 3300
actggtatag agcacctagt ccagtgacct gctgggtaaa ctgtggatga tggttgcaaa 3360
agactaattt aaaaaataac taccaagagg ccctgtctgt acctaacgcc ctatttttgc 3420
aatggctata tggcaagaaa gctggtaaac tatttgtctt tcaggacctt ttgaagtagt 3480
ttgtataact tcttaaaagt tgtgattcca gataaccagc tgtaacacag ctgagagact 3540
tttaatcaga caaagtaatt cctctcacta aactttaccc aaaaactaaa tctctaatat 3600
ggcaaaaatg gctagacacc cattttcaca ttcccatctg tcaccaattg gttaatcttt 3660
cctgatggta caggaaagct cagctactga tttttgtgat ttagaactgt atgtcagaca 3720
tccatgtttg taaaactaca catccctaat gtgtgccata gagtttaaca caagtcctgt 3780
gaatttcttc actgttgaaa attattttaa acaaaataga agctgtagta gccctttctg 3840
tgtgcacctt accaactttc tgtaaactca aaacttaaca tatttactaa gccacaagaa 3900
atttgatttc tattcaaggt ggccaaatta tttgtgtaat agaaaactga aaatctaata 3960
ttaaaaatat ggaacttcta atatattttt atatttagtt atagtttcag atatatatca 4020
tattggtatt cactaatctg ggaagggaag ggctactgca gctttacatg caatttatta 4080
aaatgattgt aaaatagctt gtatagtgta aaataagaat gatttttaga tgagattgtt 4140
ttatcatgac atgttatata ttttttgtag gggtcaaaga aatgctgatg gataacctat 4200
atgatttata gtttgtacat gcattcatac aggcagcgat ggtctcagaa accaaacagt 4260
ttgctctagg ggaagaggga gatggagact ggtcctgtgt gcagtgaagg ttgctgaggc 4320
tctgacccag tgagattaca gaggaagtta tcctctgcct cccattctga ccacccttct 4380
cattccaaca gtgagtctgt cagcgcaggt ttagtttact caatctcccc ttgcactaaa 4440
gtatgtaaag tatgtaaaca ggagacagga aggtggtgct tacatcctta aaggcaccat 4500
ctaatagcgg gttactttca catacagccc tcccccagca gttgaatgac aacagaagct 4560
tcagaagttt ggcaatagtt tgcatagagg taccagcaat atgtaaatag tgcagaatct 4620
cataggttgc caataataca ctaattcctt tctatcctac aacaagagtt tatttccaaa 4680
taaaatgagg acatgttttt gttttctttg aatgcttttt gaatgttatt tgttattttc 4740
agtattttgg agaaattatt taataaaaaa acaatcattt gctttttg 4788

<210> 2
<211> 6322
<212> DNA
<213> R. norvegicus

<220>
<221> misc_feature
<222> 24, 3663, 3680, 3684, 3685, 3791, 3805, 3806, 3813, 3854,
3861, 4162, 4177, 4205, 4206, 4240, 4246, 4247, 4262, 4283,
4284, 4293, 4295, 4311, 4354, 4358, 4359, 4360, 4398, 6010,
6011, 6013, 6014, 6065, 6069, 6145, 6161
<223> n = A,T,C or G

<400> 2
gacgctgcgg gggtggggga cctncggcgg cacggagtcc ccccccgggc tcacattaat 60
atttgccaat ggactccaaa gaatccttag ctccccctgg tagagacgaa gtccctggca 120
gtttgcttgg ccaagggagg gggagcgtaa tggactttta taaaagcctg aggggaggag 180
ctacagtcaa ggtttctgca tcttcgccct cagtggctgc tgcttctcag gcagattcca 240
agcagcagag gattctcctt gatttctcga aaggctccac aagcaatgtg cagcagcgac 300
agcagcagca gcagcagcag cagcagcagc agcagcagca gcagcagcag cagcagccag 360
gcttatccaa agccgtttca ctgtccatgg ggctgtatat gggagagaca gaaacaaaag 420
tgatggggaa tgacttgggc tacccacagc agggccaact tggcctttcc tctggggaaa 480
cagactttcg gcttctggaa gaaagcattg caaacctcaa taggtcgacc agcgttccag 540
agaaccccaa gagttcaacg tctgcaactg ggtgtgctac cccgacagag aaggagtttc 600
ccaaaactca ctcggatgca tcttcagaac agcaaaatcg aaaaagccag accggcacca 660
acggaggcag tgtgaaattg tatcccacag accaaagcac ctttgacctc ttgaaggatt 720
tggagttttc cgctgggtcc ccaagtaaag acacaaacga gagtccctgg agatcagatc 780
tgttgataga tgaaaacttg ctttctcctt tggcgggaga agatgatcca ttccttctcg 840
aagggaacac gaatgaggat tgtaagcctc ttattttacc ggacactaaa cctaaaatta 900
aggatactgg agatacaatc ttatcaagtc ccagcagtgt ggcactaccc caagtgaaaa 960
cagaaaaaga tgatttcatt gaactttgca cccccggggt aattaagcaa gagaaactgg 1020
gcccagttta ttgtcaggca agcttttctg ggacaaatat aattggtaat aaaatgtctg 1080

ccatttctgt tcatggtgtg agtacctctg gaggacagat gtaccactat gacatgaata 1140
cagcatccct ttctcagcag caggatcaga agcctgtttt taatgtcatt ccaccaattc 1200
ctgttggttc tgaaaactgg aataggtgcc aaggctccgg agaggacagc ctgacttcct 1260
tgggggctct gaacttccca ggccggtcag tgttttctaa tgggtactca agccctggaa 1320
tgagaccaga tgtaagctct cctccatcca gctcgtcagc agccacggga ccacctccca 1380
agctctgcct ggtgtgctcc gatgaagctt caggatgtca ttacggggtg ctgacatgtg 1440
gaagctgcaa agtattcttt aaaagagcag tggaaggaca gcacaattac ctttgtgctg 1500
gaagaaacga ttgcatcatt gataaaattc gaaggaaaaa ctgcccagca tgccgctatc 1560
ggaaatgtct tcaggctgga atgaaccttg aagctcgaaa aacaaagaaa aaaatcaaag 1620
ggattcagca agccactgca ggagtctcac aagacacttc ggaaaatcct aacaaaacaa 1680
tagttcctgc agcattacca cagctcaccc ctaccttggt gtcactgctg gaggtgattg 1740
aacccgaggt gttgtatgca ggatatgata gctctgttcc agattcagca tggagaatta 1800
tgaccacact caacatgtta ggtgggcgtc aagtgattgc agcagtgaaa tgggcaaagg 1860
cgatactagg cttgagaaac ttacacctcg atgaccaaat gaccctgcta cagtactcat 1920
ggatgtttct catggcattt gccttgggtt ggagatcata cagacaatca agcggaaacc 1980
tgctctgctt tgctcctgat ctgattatta atgagcagag aatgtctcta ccctgcatgt 2040
atgaccaatg taaacacatg ctgtttgtct cctctgaatt acaaagattg caggtatcct 2100
atgaagagta tctctgtatg aaaaccttac tgcttctctc ctcagttcct aaggaaggtc 2160
tgaagagcca agagttattt gatgagattc gaatgactta tatcaaagag ctaggaaaag 2220
ccatcgtcaa aagggaaggg aactccagtc agaactggca acggttttac caactgacaa 2280
agcttctgga ctccatgcat gaggtggttg agaatctcct tacctactgc ttccagacat 2340
ttttggataa gaccatgagt attgaattcc cagagatgtt agctgaaatc atcactaatc 2400
agataccaaa atattcaaat ggaaatatca aaaagcttct gtttcatcaa aaatgactgc 2460
cttactaaga aaggttgcct taaagaaagt tgaatttata gcttttactg tacaaactta 2520
tcaatttgtc ttgtagatgt tttgttgttc tttttgtttc tgtcttgttt tgttttaaac 2580
acgcagtaca tgtggtttat agagggccaa gacttggcga cagaagcagt tgagtcaaca 2640
ctctgaagtg atgacacagc acacagtgaa gtgtattgtt ggtgtatcac agaaactaac 2700
agttacgtgg aggcatggcc actgtcagag agggaccgca cctaaaccac cgtgcccaag 2760
tccatgtggt tcaactttct gactcagaac tttacagttg gctgggtaaa actttctaga 2820
ctttctgttg gtgtattttt cccatgtata gttaggatgg tattttgatt tatgcatgca 2880
aacctgaaaa aagtttacaa gtgtatatca gaaaagggaa gttgtgcctt ttatagctat 2940
tactgtctgg ttttaacaat ttcctttata ttcagtgaac tatgcttgct cgtttctctt 3000
caataatttt tgtattccag ttattgtaca gctgtttaag atgggcagct gcttcacagc 3060
tttcctagac gctaacatta atttccgtgt gaaaatgggt cggtgcttct accctgttgg 3120
caccagctat cagaagacca cagaaattga ctcagatctc cagtattctt gttaaaaagc 3180
tcttactctg tatatatctg cttccatgga gaattacata ggctgagcag attacatagg 3240
ctgagcagat taaccgtcct aactggtgta gagcacctag tccagtgacc ttctgggtaa 3300
accgtggatg atggttacag aagactggtg ggaaaacagt aactaccaaa aggccccttt 3360
ccatctaatg caccatctct tcaatgggga gatagcaacc aagcccgtaa atcagctctt 3420
tcaggacctt ctggagtggt ttgcataaca ttttaaaatg tattattcca gatagccagc 3480
tctgataaag ccgagagatt gtttaatcag accaagtaac ttctctcatt aaacttaccc 3540
ccaactaaat cgctaataca gcaagaatgg ctagacaccc attttcacat ctcacccgca 3600
ccgattggtc tagctctcat ggtggtcagg agaatcagct actgattttt gttacttaga 3660
atnttcagga ctcgcatttn tccnnctaca catccctaca tgtgccatag aatttaacac 3720
aagtcctgtg aacttcttca cattgagaat tatcatttta aacaaaacag aagcagtagt 3780
agccctttct ntgtgcacct taccnncttt ctntgactca aagcttaata tgcttactaa 3840
gccacaagaa atcngatttc nacttaaagg cgccaaatta tttgtgtaat agaaaaactg 3900
aaaatctaat attaaaaata tgaaacttct aatatatttt tatatttagt tatagtttcg 3960
atatatatca tatcggtatt cactgatctt gggaaaggga aagggctact gcagctttac 4020
atgcaattta ttaactgact gtaaaatagc tgtatagtaa taagaatgac ttttagtgag 4080
attgctttat catgacatgt tatatatttt tcgtaggggt caaagaaata ttgatggata 4140
tgatagccta tatgatttaa tngtatataa aagcatncaa acaggcctta acgcgtcttg 4200
gaaannaaaa tacctttgtt ctaagctagg gaagggagcn ggagannggc cccgtgtgta 4260
tnggaggttc cgaggctcgg atnnaagaga tcnanagggg atctaattcc ntacctccat 4320
ctaattacct caccacccat gatcctgtca gtgnaggnnn ggttattaaa tcccccgtta 4380
tactaatata aatagganag aagggtggcg ctcacgtctg ttccaggcgc cgcagtagca 4440
gggttatttt ccatgcagcc tcccgacaag gttagcagag ggaggctttg gcaagtttgg 4500
cgtggcgtgc atagaggcac cagcaacatg taaacctaaa gagcccatag gaagccaaga 4560
atacactaat cctccccacc cttcaatagt ccatttccaa gtaagatgag gacatgctta 4620
tgttttcttt gaatgctttt agaatgttgt tattttcagt attttgcaga aattatttaa 4680
taaaaaagta taatttgaat tctctctaaa agggattgtt cagtttgtaa tggtttaaat 4740
tggtctcaaa gtactttaag ataattgtaa cccagctgga tgtgaaattt atggtgccta 4800
agaaatacca cttgaatatt atcaagacag tgttaagttt taaaatgagc ttctcaaaaa 4860
tagattattg tacatttatg gaatgttata tggttaaacc caaaaaagca catcacacat 4920
aaatctgctt tcagcttggc tttcaaaaat agagctccaa aaacgaaaaa ggagaagaaa 4980
aagtatatat atgcgttgtt attaacagaa ggcaacagac attcataaaa ctactaccga 5040
agctttcctt gaagcgtata aagagccatg ctcctttagt atgtggggaa gaagagagcc 5100

gtcatagttt cgagtacaga gagaagatgc ggtactgtct ccgtgtgtgg cttcataccg 5160
ttcctaacta tttaggttta taataacttc agtgagactc ggtgacatgc ctgtatgact 5220
catgaccgat cttgaaagat atctttaatt actggtagga caaaagggac actctggtta 5280
ttttaggcct tggcttggga tactgtatat ccagaagaaa ggagacagga aacttgggga 5340
agggaaggga acctaggaag cactgccttc tgtaggaaag aacacaccaa taagtgagag 5400
tacccaaagg gacaaggcca cacagtgtgg ggtctaagga tgagtcaggg tgagctctgg 5460
tgggcatgga gaagccagca actccagtgc tacagagcag ggcagggcag ggatgggaca 5520
agatggatgc ggatcccagt cccagtagtt tgctccctct tatttaccat gggatgaacc 5580
atggagtatt gatctgtcag cactcaagga tcatggagct tgagattccg gttggtcacc 5640
ccaacggtaa gctgagattg aatgtgtttc ttatgtgccg gtttcagtgt tagaaggcga 5700
aacagagtgt acagaagaca ctgcaaaccg gtcagatgaa agtcttctca ttcccaaact 5760
attttcagtc agcctgctct atcaggactg gtgaccagct gctaggacag ggtcggcgct 5820
tctgtctaga atatgcctga aaggatttta ttttctgata aatggctgta tgaaaatacc 5880
ctcctcaata acctgcttaa ctacatagag atttcagtgt gtcaatattc tattttgtat 5940
attaaacaaa ggctatataa tggggacaaa tctatattat actgtgtatg gcattattaa 6000
gaagcttttn nannattttt tatcacagta atttttaaat gtgtaaaaaa ttaaaaatta 6060
gtgantccng tttaaaaata aaagttgtag ttttttattc atgctgaata acctgtagtt 6120
taaaaatccg tctttctacc tacanagtga aatgtcagac ngtaaaattt tgtgtggaaa 6180
tgtttaactt ttatttttct ttaaatttgc tgtcttggta ttaccaaacc acacattgta 6240
ctgaattggc agtaaatgtt agtcagccat ttacagcaat gccaaatatg gataaacatc 6300
ataataaaat atctgctttt tc 6322

<210> 3
<211> 2575
<212> DNA
<213> M. musculus

<400> 3
ggaagttaat atttgccaat ggactccaaa gaatccttag ctccccctgg tagagacgaa 60
gtccccagca gtttgcttgg ccgggggagg ggaagcgtga tggacttgta taaaaccctg 120
aggggtggag ctacagtcaa ggtttctgcg tcttcaccct cagtggctgc tgcttctcag 180
gcagattcca agcagcagag gattctcctt gatttttcaa aaggctcagc aagcaatgca 240
cagcagcagc agcagcagca gcagccgcag ccagatttat ccaaagccgt ttcactgtcc 300
atgggactgt atatgggaga gaccgaaaca aaagtgatgg ggaatgactt gggctaccca 360
cagcagggcc agcttggcct ctcctctggg gaaacagact ttcggcttct ggaagaaagc 420
attgcaaacc tcaataggtc gaccagccgt ccagagaatc ccaagagttc aacacctgca 480
gctgggtgtg ctaccccgac agagaaggag tttccccaga ctcactctga tccatcttca 540
gaacagcaaa atagaaaaag ccagcctggc accaacggtg gcagtgtgaa attgtatacc 600
acagaccaaa gcacctttga catcttgcag gatttggagt tttctgccgg gtccccaggt 660
aaagagacaa acgagagtcc ttggaggtca gacctgttga tagatgaaaa cttgctttct 720
cctttggcgg gagaagatga tccattcctt ctggaagggg acgtgaatga ggattgcaag 780
cctcttattt taccggacac taaacctaaa attcaggata ctggagatac aatcttatca 840
agccccagca gtgtggcact gccccaagtg aaaacagaga aagatgattt cattgagctt 900
tgcacccctg gggtaattaa gcaagagaaa ctgggcccgg tttattgcca ggcaagcttt 960
tctgggacaa atataattgg gaataaaatg tctgccattt ctgttcatgg cgtgagtacc 1020
tctggaggac agatgtacca ctatgacatg aatacagcat ccctttctca gcagcaggat 1080
cagaagcctg tttttaatgt cattccacca attcctgttg gttctgaaaa ctggaatagg 1140
tgccaagggt ctggagagga caacctgact tccttggggg ctatgaactt cgcaggccgc 1200
tcagtgtttt ctaatggata ttcaagccct ggaatgagac cagatgtgag ttctcctccg 1260
tccagctcct ccacagcaac gggaccacct cccaaactct gcctggtgtg ctccgatgaa 1320
gcttcggtat gccattatgg ggtgctgacg tgtggaagct gtaaagtctt ctttaaaaga 1380
gcagtggaag gacagcacaa ttacctttgt gctggaagaa atgattgcat cattgataaa 1440
attcgaagaa aaaactgtcc agcatgccgc tatcgaaaat gtcttcaagc tggaatgaac 1500
ctggaagctc gaaaaacgaa gaaaaaaatt aaaggaattc agcaagccac tgcaggagtc 1560
tcacaagaca cttctgaaaa cgctaacaaa acaatagttc ctgccgcgct gccacagctt 1620
acccctaccc tggtgtcact gctggaggtg atcgagcctg aggtgttata tgcaggatat 1680
gacagctctg ttccagactc agcatggaga attatgacca cgctcaacat gttaggtggg 1740
cgccaagtga ttgccgcagt gaaatgggca aaggcgatac caggattcag aaacttacac 1800
ctggatgacc aaatgaccct tctacagtac tcatggatgt ttctcatggc atttgccctg 1860
ggttggagat catacagaca agcaagtgga aacctgctat gctttgctcc tgatctgatt 1920
attaatgagc agagaatgac tctaccctgc atgtatgacc aatgtaaaca catgctgttt 1980
atctccactg aattacaaag attgcaggta tcctatgaag agtatctctg tatgaaaacc 2040
ttactgcttc tctcctcagt tcctaaggaa ggtctgaaga gccaagagtt atttgatgag 2100
attcgaatga cttatatcaa agagctagga aaagccattg tcaaaaggga aggaaactcc 2160
agtcagaatt ggcagcggtt ttatcaactg acaaaacttt tggactccat gcatgatgtg 2220

gttgaaaatc tccttagcta ctgcttccaa acatttttgg ataagtccat gagtattgaa 2280
ttcccagaga tgttagctga aatcatcact aatcagatac caaaatactc aaatggaaat 2340
atcaaaaagc ttctgtttca tcagaaatga ctgccttact aagaaaggct gccttaaaga 2400
aagttgaatt tatagctttt actgtacaaa cttatcaact tgtcttgtag atgttttgtc 2460
gttctttttg tttgtcttgt ttgttttcta tacgcactac atgtggtctc tagagggcca 2520
agacttggca acagaagcag atgagccatc acttttcagt gacaggaaag cagac 2575

<210> 4
<211> 20
<212> DNA
<213> Artificial Sequence

<220>
<223> Oligomeric compound

<400> 4
cgtgtgtctg tgctagtccc 20

<210> 5
<211> 20
<212> DNA
<213> Artificial Sequence

<220>
<223> Oligomeric compound

<400> 5
ggcaacgtga acaggtccaa 20

<210> 6
<211> 18
<212> DNA
<213> Artificial Sequence

<220>
<223> Oligomeric compound

<400> 6
gcccattgct ggacatgc 18

<210> 7
<211> 20
<212> DNA
<213> Artificial Sequence

<220>
<223> Oligomeric compound

<400> 7
agcccattgc tggacatgca 20

<210> 8
<211> 20
<212> DNA
<213> Artificial Sequence

<220>
<223> Oligomeric compound

<400> 8
ttgtcccagt cccaggcctc 20

<210> 9
<211> 20
<212> DNA
<213> Artificial Sequence

<220>
<223> Oligomeric compound

<400> 9
ctttccgttg gacccctggg                                                    20

<210> 10
<211> 20
<212> DNA
<213> Artificial Sequence

<220>
<223> Oligomeric compound

<400> 10
gtgcgcgcga gcccgaaatc                                                    20

<210> 11
<211> 20
<212> DNA
<213> Artificial Sequence

<220>
<223> Oligomeric compound

<400> 11
atccaagtgc tactgtagta                                                    20

<210> 12
<211> 20
<212> DNA
<213> Artificial Sequence

<220>
<223> Oligomeric compound

<220>
<221> misc_feature
<222> 1, 2, 3, 4, 5, 6, 7, 8, 9, 10, 11, 12, 13, 14, 15, 16, 17,
18, 19, 20
<223> n = A,T,C or G

<400> 12
nnnnnnnnnn nnnnnnnnnn                                                    20

<210> 13
<211> 20
<212> DNA
<213> Artificial Sequence

<220>
<223> Oligomeric compound

<400> 13
gccctccatg ctggcacagg                                                    20

<210> 14
<211> 20
<212> DNA
<213> Artificial Sequence

<220>
<223> Oligomeric compound

<400> 14
agcaaaagat caatccgtta                                                    20

<210> 15
<211> 20
<212> DNA
<213> Artificial Sequence

<220>
<223> Oligomeric compound

<400> 15
tacagaaggc tgggccttga                                                    20

<210> 16
<211> 20
<212> DNA
<213> Artificial Sequence

<220>
<223> Oligomeric compound

<400> 16
atgcattctg cccccaagga                                                    20

<210> 17
<211> 22
<212> DNA
<213> Artificial Sequence

<220>
<223> PCR primer

<400> 17
ttgacatttt gcaggatttg ga                                                 22

<210> 18
<211> 24
<212> DNA
<213> Artificial Sequence

<220>
<223> PCR primer

<400> 18
ccaaggactc tcattcgtct cttt                                               24

<210> 19
<211> 16
<212> DNA
<213> Artificial Sequence

<220>
<223> PCR probe

<400> 19
tttcttctgg gtcccc                                                        16

<210> 20
<211> 25
<212> DNA
<213> Artificial Sequence

<220>
<223> PCR primer

<400> 20
aaacaatagt tcctgcagca ttacc                                              25

<210> 21

<211> 23
<212> DNA
<213> Artificial Sequence

<220>
<223> PCR primer

<400> 21
catacaacac ctcgggttca atc                                              23

<210> 22
<211> 23
<212> DNA
<213> Artificial Sequence

<220>
<223> PCR probe

<400> 22
acccctacct tggtgtcact gct                                              23

<210> 23
<211> 23
<212> DNA
<213> Artificial Sequence

<220>
<223> PCR primer

<400> 23
gacatcttgc aggatttgga gtt                                              23

<210> 24
<211> 23
<212> DNA
<213> Artificial Sequence

<220>
<223> PCR primer

<400> 24
aacaggtctg acctccaagg act                                              23

<210> 25
<211> 27
<212> DNA
<213> Artificial Sequence

<220>
<223> PCR probe

<400> 25
cgggtcccca ggtaaagaga caaacga                                          27

<210> 26
<211> 20
<212> DNA
<213> Artificial Sequence

<220>
<223> Oligomeric compound

<400> 26
tctgtctctc ccatatacag                                                  20

<210> 27
<211> 20

<212> DNA
<213> Artificial Sequence

<220>
<223> Oligomeric compound

<400> 27
tgtttctgtc tctcccatat                                                    20

<210> 28
<211> 20
<212> DNA
<213> Artificial Sequence

<220>
<223> Oligomeric compound

<400> 28
cttttgtttc tgtctctccc                                                    20

<210> 29
<211> 20
<212> DNA
<213> Artificial Sequence

<220>
<223> Oligomeric compound

<400> 29
atcacttttg tttctgtctc                                                    20

<210> 30
<211> 20
<212> DNA
<213> Artificial Sequence

<220>
<223> Oligomeric compound

<400> 30
gtttgcaatg ctttcttcca                                                    20

<210> 31
<211> 20
<212> DNA
<213> Artificial Sequence

<220>
<223> Oligomeric compound

<400> 31
tgaggtttgc aatgctttct                                                    20

<210> 32
<211> 20
<212> DNA
<213> Artificial Sequence

<220>
<223> Oligomeric compound

<400> 32
ctattgaggt ttgcaatgct                                                    20

<210> 33
<211> 20
<212> DNA

<213> Artificial Sequence

<220>
<223> Oligomeric compound

<400> 33
cgacctattg aggtttgcaa                                                    20

<210> 34
<211> 20
<212> DNA
<213> Artificial Sequence

<220>
<223> Oligomeric compound

<400> 34
ctggtcgacc tattgaggtt                                                    20

<210> 35
<211> 20
<212> DNA
<213> Artificial Sequence

<220>
<223> Oligomeric compound

<400> 35
ctgtggtata caatttcaca                                                    20

<210> 36
<211> 20
<212> DNA
<213> Artificial Sequence

<220>
<223> Oligomeric compound

<400> 36
ctttggtctg tggtatacaa                                                    20

<210> 37
<211> 20
<212> DNA
<213> Artificial Sequence

<220>
<223> Oligomeric compound

<400> 37
gtcaaaggtg ctttggtctg                                                    20

<210> 38
<211> 20
<212> DNA
<213> Artificial Sequence

<220>
<223> Oligomeric compound

<400> 38
ggtttagtgt ccggtaaaat                                                    20

<210> 39
<211> 20
<212> DNA
<213> Artificial Sequence

<220>
<223> Oligomeric compound

<400> 39
ctttttctgt tttcacttgg                                                   20

<210> 40
<211> 20
<212> DNA
<213> Artificial Sequence

<220>
<223> Oligomeric compound

<400> 40
ttctcttgct taattacccc                                                   20

<210> 41
<211> 20
<212> DNA
<213> Artificial Sequence

<220>
<223> Oligomeric compound

<400> 41
cagtttctct tgcttaatta                                                   20

<210> 42
<211> 20
<212> DNA
<213> Artificial Sequence

<220>
<223> Oligomeric compound

<400> 42
gcccagtttc tcttgcttaa                                                   20

<210> 43
<211> 20
<212> DNA
<213> Artificial Sequence

<220>
<223> Oligomeric compound

<400> 43
tttattacca attatatttg                                                   20

<210> 44
<211> 20
<212> DNA
<213> Artificial Sequence

<220>
<223> Oligomeric compound

<400> 44
acattttatt accaattata                                                   20

<210> 45
<211> 20
<212> DNA
<213> Artificial Sequence

<220>
<223> Oligomeric compound

<400> 45
gcagacattt tattaccaat                                                    20

<210> 46
<211> 20
<212> DNA
<213> Artificial Sequence

<220>
<223> Oligomeric compound

<400> 46
aatggcagac attttattac                                                    20

<210> 47
<211> 20
<212> DNA
<213> Artificial Sequence

<220>
<223> Oligomeric compound

<400> 47
cagaaatggc agacatttta                                                    20

<210> 48
<211> 20
<212> DNA
<213> Artificial Sequence

<220>
<223> Oligomeric compound

<400> 48
tgaacagaaa tggcagacat                                                    20

<210> 49
<211> 20
<212> DNA
<213> Artificial Sequence

<220>
<223> Oligomeric compound

<400> 49
ccatgaacag aaatggcaga                                                    20

<210> 50
<211> 20
<212> DNA
<213> Artificial Sequence

<220>
<223> Oligomeric compound

<400> 50
cacaccatga acagaaatgg                                                    20

<210> 51
<211> 20
<212> DNA
<213> Artificial Sequence

<220>

<223> Oligomeric compound

<400> 51
tactcacacc atgaacagaa                                                   20

<210> 52
<211> 20
<212> DNA
<213> Artificial Sequence

<220>
<223> Oligomeric compound

<400> 52
gaggtactca caccatgaac                                                   20

<210> 53
<211> 20
<212> DNA
<213> Artificial Sequence

<220>
<223> Oligomeric compound

<400> 53
tccagaggta ctcacaccat                                                   20

<210> 54
<211> 20
<212> DNA
<213> Artificial Sequence

<220>
<223> Oligomeric compound

<400> 54
gtcctccaga ggtactcaca                                                   20

<210> 55
<211> 20
<212> DNA
<213> Artificial Sequence

<220>
<223> Oligomeric compound

<400> 55
atctgtcctc cagaggtact                                                   20

<210> 56
<211> 20
<212> DNA
<213> Artificial Sequence

<220>
<223> Oligomeric compound

<400> 56
gtacatctgt cctccagagg                                                   20

<210> 57
<211> 20
<212> DNA
<213> Artificial Sequence

<220>
<223> Oligomeric compound

<400> 57
agtggtacat ctgtcctcca                                                    20

<210> 58
<211> 20
<212> DNA
<213> Artificial Sequence

<220>
<223> Oligomeric compound

<400> 58
tcatagtggt acatctgtcc                                                    20

<210> 59
<211> 20
<212> DNA
<213> Artificial Sequence

<220>
<223> Oligomeric compound

<400> 59
catgtcatag tggtacatct                                                    20

<210> 60
<211> 20
<212> DNA
<213> Artificial Sequence

<220>
<223> Oligomeric compound

<400> 60
tattcatgtc atagtggtac                                                    20

<210> 61
<211> 20
<212> DNA
<213> Artificial Sequence

<220>
<223> Oligomeric compound

<400> 61
gctgtattca tgtcatagtg                                                    20

<210> 62
<211> 20
<212> DNA
<213> Artificial Sequence

<220>
<223> Oligomeric compound

<400> 62
ggatgctgta ttcatgtcat                                                    20

<210> 63
<211> 20
<212> DNA
<213> Artificial Sequence

<220>
<223> Oligomeric compound

<400> 63
aaagggatgc tgtattcatg                                                    20

<210> 64
<211> 20
<212> DNA
<213> Artificial Sequence

<220>
<223> Oligomeric compound

<400> 64
tgagaaaggg atgctgtatt                                                    20

<210> 65
<211> 20
<212> DNA
<213> Artificial Sequence

<220>
<223> Oligomeric compound

<400> 65
tggtggaatg acattaaaaa                                                    20

<210> 66
<211> 20
<212> DNA
<213> Artificial Sequence

<220>
<223> Oligomeric compound

<400> 66
gaattggtgg aatgacatta                                                    20

<210> 67
<211> 20
<212> DNA
<213> Artificial Sequence

<220>
<223> Oligomeric compound

<400> 67
gagcttacat ctggtctcat                                                    20

<210> 68
<211> 20
<212> DNA
<213> Artificial Sequence

<220>
<223> Oligomeric compound

<400> 68
aggagagctt acatctggtc                                                    20

<210> 69
<211> 20
<212> DNA
<213> Artificial Sequence

<220>
<223> Oligomeric compound

<400> 69

atggaggaga gcttacatct                                                    20

<210> 70
<211> 20
<212> DNA
<213> Artificial Sequence

<220>
<223> Oligomeric compound

<400> 70
ctggatggag gagagcttac                                                    20

<210> 71
<211> 20
<212> DNA
<213> Artificial Sequence

<220>
<223> Oligomeric compound

<400> 71
gagctggatg gaggagagct                                                    20

<210> 72
<211> 20
<212> DNA
<213> Artificial Sequence

<220>
<223> Oligomeric compound

<400> 72
tgtccttcca ctgctctttt                                                    20

<210> 73
<211> 20
<212> DNA
<213> Artificial Sequence

<220>
<223> Oligomeric compound

<400> 73
gtgctgtcct tccactgctc                                                    20

<210> 74
<211> 20
<212> DNA
<213> Artificial Sequence

<220>
<223> Oligomeric compound

<400> 74
aattgtgctg tccttccact                                                    20

<210> 75
<211> 20
<212> DNA
<213> Artificial Sequence

<220>
<223> Oligomeric compound

<400> 75
aggtaattgt gctgtccttc                                                    20

<210> 76
<211> 20
<212> DNA
<213> Artificial Sequence

<220>
<223> Oligomeric compound

<400> 76
cggcatgctg ggcagttttt                                                    20

<210> 77
<211> 20
<212> DNA
<213> Artificial Sequence

<220>
<223> Oligomeric compound

<400> 77
atagcggcat gctgggcagt                                                    20

<210> 78
<211> 20
<212> DNA
<213> Artificial Sequence

<220>
<223> Oligomeric compound

<400> 78
cgatagcggc atgctgggca                                                    20

<210> 79
<211> 20
<212> DNA
<213> Artificial Sequence

<220>
<223> Oligomeric compound

<400> 79
attccagcct gaagacattt                                                    20

<210> 80
<211> 20
<212> DNA
<213> Artificial Sequence

<220>
<223> Oligomeric compound

<400> 80
gttcattcca gcctgaagac                                                    20

<210> 81
<211> 20
<212> DNA
<213> Artificial Sequence

<220>
<223> Oligomeric compound

<400> 81
ttctttgttt ttcgagcttc                                                    20

<210> 82
<211> 20
<212> DNA
<213> Artificial Sequence

<220>
<223> Oligomeric compound

<400> 82
ttttttcttt gtttttcgag                                                    20

<210> 83
<211> 20
<212> DNA
<213> Artificial Sequence

<220>
<223> Oligomeric compound

<400> 83
caggaactat tgttttgtta                                                    20

<210> 84
<211> 20
<212> DNA
<213> Artificial Sequence

<220>
<223> Oligomeric compound

<400> 84
tgcaggaact attgttttgt                                                    20

<210> 85
<211> 20
<212> DNA
<213> Artificial Sequence

<220>
<223> Oligomeric compound

<400> 85
gagctatcat atcctgcata                                                    20

<210> 86
<211> 20
<212> DNA
<213> Artificial Sequence

<220>
<223> Oligomeric compound

<400> 86
aacagagcta tcatatcctg                                                    20

<210> 87
<211> 20
<212> DNA
<213> Artificial Sequence

<220>
<223> Oligomeric compound

<400> 87
ctggaacaga gctatcatat                                                    20

<210> 88

<211> 20
<212> DNA
<213> Artificial Sequence

<220>
<223> Oligomeric compound

<400> 88
ttcactgctg caatcacttg                                                    20

<210> 89
<211> 20
<212> DNA
<213> Artificial Sequence

<220>
<223> Oligomeric compound

<400> 89
ccatttcact gctgcaatca                                                    20

<210> 90
<211> 20
<212> DNA
<213> Artificial Sequence

<220>
<223> Oligomeric compound

<400> 90
ttgcccattt cactgctgca                                                    20

<210> 91
<211> 20
<212> DNA
<213> Artificial Sequence

<220>
<223> Oligomeric compound

<400> 91
ataatcagat caggagcaaa                                                    20

<210> 92
<211> 20
<212> DNA
<213> Artificial Sequence

<220>
<223> Oligomeric compound

<400> 92
attaataatc agatcaggag                                                    20

<210> 93
<211> 20
<212> DNA
<213> Artificial Sequence

<220>
<223> Oligomeric compound

<400> 93
gctcattaat aatcagatca                                                    20

<210> 94
<211> 20

<212> DNA
<213> Artificial Sequence

<220>
<223> Oligomeric compound

<400> 94
ctctgctcat taataatcag                                                    20

<210> 95
<211> 20
<212> DNA
<213> Artificial Sequence

<220>
<223> Oligomeric compound

<400> 95
cattctctgc tcattaataa                                                    20

<210> 96
<211> 20
<212> DNA
<213> Artificial Sequence

<220>
<223> Oligomeric compound

<400> 96
agcatgtgtt tacattggtc                                                    20

<210> 97
<211> 20
<212> DNA
<213> Artificial Sequence

<220>
<223> Oligomeric compound

<400> 97
aaggttttca tacagagata                                                    20

<210> 98
<211> 20
<212> DNA
<213> Artificial Sequence

<220>
<223> Oligomeric compound

<400> 98
cagtaaggtt ttcatacaga                                                    20

<210> 99
<211> 20
<212> DNA
<213> Artificial Sequence

<220>
<223> Oligomeric compound

<400> 99
gaagcagtaa ggttttcata                                                    20

<210> 100
<211> 20
<212> DNA

<213> Artificial Sequence

<220>
<223> Oligomeric compound

<400> 100
gagagaagca gtaaggtttt                                                   20

<210> 101
<211> 20
<212> DNA
<213> Artificial Sequence

<220>
<223> Oligomeric compound

<400> 101
gcttttccta gctctttgat                                                   20

<210> 102
<211> 20
<212> DNA
<213> Artificial Sequence

<220>
<223> Oligomeric compound

<400> 102
atggcttttc ctagctcttt                                                   20

<210> 103
<211> 20
<212> DNA
<213> Artificial Sequence

<220>
<223> Oligomeric compound

<400> 103
atggtcttat ccaaaaatgt                                                   20

<210> 104
<211> 20
<212> DNA
<213> Artificial Sequence

<220>
<223> Oligomeric compound

<400> 104
actcatggtc ttatccaaaa                                                   20

<210> 105
<211> 20
<212> DNA
<213> Artificial Sequence

<220>
<223> Oligomeric compound

<400> 105
caatactcat ggtcttatcc                                                   20

<210> 106
<211> 20
<212> DNA
<213> Artificial Sequence

<220>
<223> Oligomeric compound

<400> 106
aattcaatac tcatggtctt                                                    20

<210> 107
<211> 20
<212> DNA
<213> Artificial Sequence

<220>
<223> Oligomeric compound

<400> 107
atgatttcag ctaacatctc                                                    20

<210> 108
<211> 20
<212> DNA
<213> Artificial Sequence

<220>
<223> Oligomeric compound

<400> 108
gtgatgattt cagctaacat                                                    20

<210> 109
<211> 20
<212> DNA
<213> Artificial Sequence

<220>
<223> Oligomeric compound

<400> 109
gaatattttg gtatctgatt                                                    20

<210> 110
<211> 20
<212> DNA
<213> Artificial Sequence

<220>
<223> Oligomeric compound

<400> 110
atttgaatat tttggtatct                                                    20

<210> 111
<211> 20
<212> DNA
<213> Artificial Sequence

<220>
<223> Oligomeric compound

<400> 111
ttccatttga atattttggt                                                    20

<210> 112
<211> 20
<212> DNA
<213> Artificial Sequence

<220>
<223> Oligomeric compound

<400> 112
atatttccat ttgaatattt                                                    20

<210> 113
<211> 20
<212> DNA
<213> Artificial Sequence

<220>
<223> Oligomeric compound

<400> 113
tttttgatat ttccatttga                                                    20

<210> 114
<211> 20
<212> DNA
<213> Artificial Sequence

<220>
<223> Oligomeric compound

<400> 114
ccttccctga aggttcctcc                                                    20